(19) Europäisches Patentamt
European Patent Office
Office européen des brevets

(11) EP 4 707 386 A1

(12) EUROPEAN PATENT APPLICATION
published in accordance with Art. 153(4) EPC

(43) Date of publication:
**11.03.2026 Bulletin 2026/11**

(21) Application number: **24800102.6**

(22) Date of filing: **26.04.2024**

(51) International Patent Classification (IPC):
***C12N 7/00*** *(2006.01)* ***A61K 9/51*** *(2006.01)*
***A61K 35/76*** *(2015.01)* ***A61P 35/00*** *(2006.01)*
***C12N 15/40*** *(2006.01)*

(52) Cooperative Patent Classification (CPC):
**A61K 9/51; A61K 35/76; A61P 35/00; C12N 7/00;**
C07K 14/08

(86) International application number:
**PCT/JP2024/016503**

(87) International publication number:
**WO 2024/228371 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.05.2023 JP 2023076053**

(71) Applicants:
- **AGC INC.**
**Chiyoda-ku,**
**Tokyo 1008405 (JP)**
- **The University of Tokyo**
**Bunkyo-ku, Tokyo 113-8654 (JP)**
- **National University Corporation Tottori University**
**Tottori-shi**
**Tottori 680-8550 (JP)**

(72) Inventors:
- **OTA, Yu**
**Tokyo 100-8405 (JP)**
- **OKAZOE, Takashi**
**Tokyo 100-8405 (JP)**
- **AIKAWA, Kohsuke**
**Tokyo 113-8654 (JP)**
- **SANDO, Shinsuke**
**Tokyo 113-8654 (JP)**
- **MORIMOTO, Jumpei**
**Tokyo 113-8654 (JP)**
- **MATSUURA, Kazunori**
**Tottori-shi, Tottori 680-8550 (JP)**
- **FURUKAWA, Hiroto**
**Tottori-shi, Tottori 680-8550 (JP)**

(74) Representative: **Müller-Boré & Partner Patentanwälte PartG mbB**
**Friedenheimer Brücke 21**
**80639 München (DE)**

Remarks:
The complete document including Reference Table(s) and the Sequence Listing(s) can be downloaded from the EPO website

(54) **ARTIFICIAL VIRUS CAPSID**

(57) The present invention provides an artificial viral capsid modified with a compound containing a fluorine atom. The artificial viral capsid is formed by self-assembly of multiple subunits, wherein the subunit comprises a β-annulus peptide of tomato bushy stunt virus, a group derived from a fluorine-containing compound, and a divalent linking group that links the β-annulus peptide to the group derived from a fluorine-containing compound; and the divalent linking group is linked to a C-terminus of the β-annulus peptide.

EP 4 707 386 A1

FIG. 14

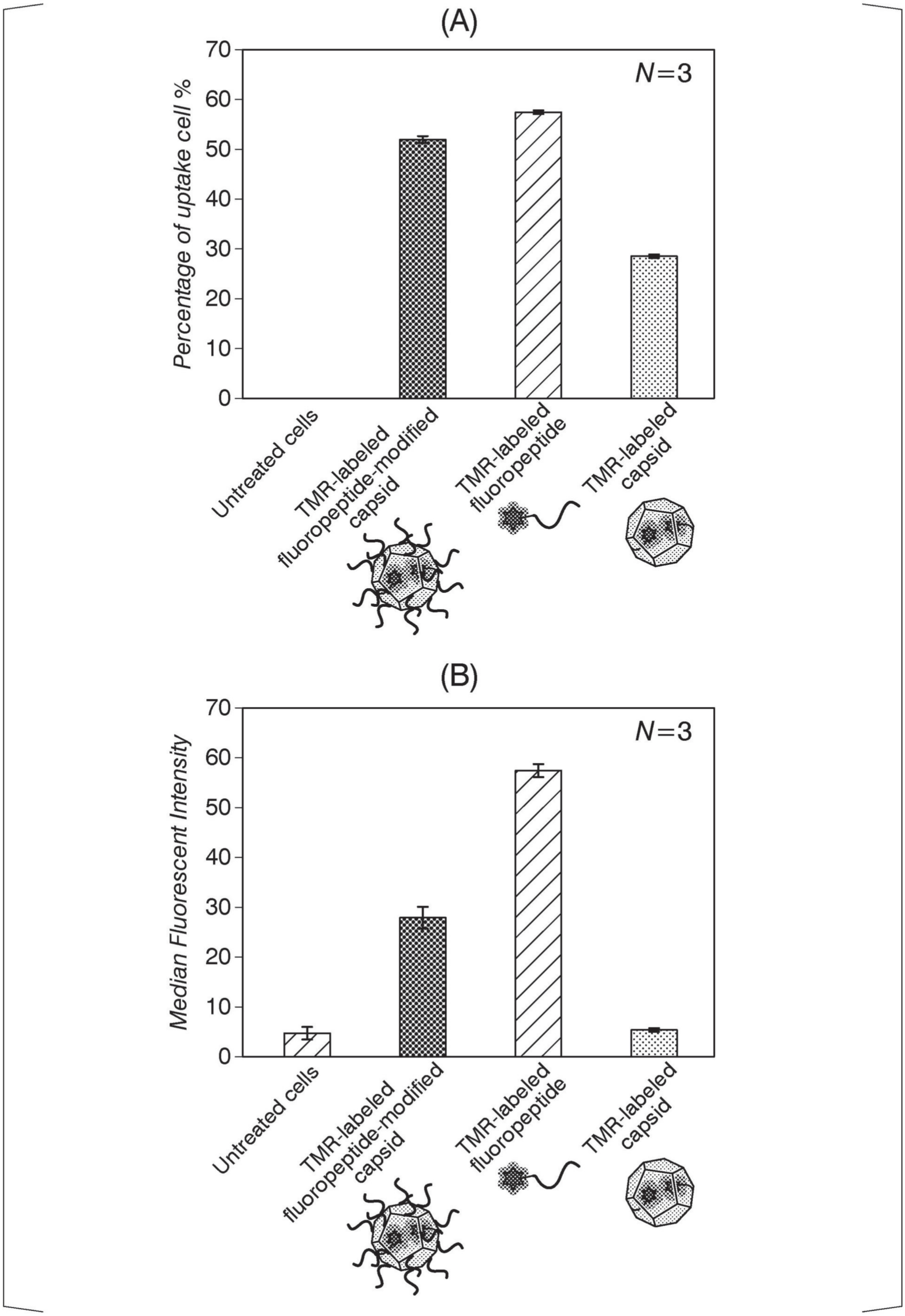
(A)
N=3
Percentage of uptake cell %
0
10
20
30
40
50
60
70
Untreated cells
TMR-labeled fluoropeptide-modified capsid
TMR-labeled fluoropeptide
TMR-labeled capsid
(B)
N=3
Median Fluorescent Intensity
0
10
20
30
40
50
60
70
Untreated cells
TMR-labeled fluoropeptide-modified capsid
TMR-labeled fluoropeptide
TMR-labeled capsid

## Description

TECHNICAL FIELD

The present invention relates to an artificial viral capsid modified with a compound containing a fluorine atom.

This application claims priority based on Japanese Patent Application No. 2023-076053, filed on May 2, 2023, the contents of which are incorporated herein by reference.

BACKGROUND ART

Spherical artificial viral capsids can be created by in vitro self-assembly of β-annulus peptides derived from proteins that form the dodecahedral internal skeleton of the capsid of tomato bushy stunt virus (Non-Patent Documents 1-4). The β-annulus peptide is a 24-amino acid long peptide, which is the smallest unit corresponding to a portion of the 388-amino acid capsid protein. The β-annulus peptides spontaneously self-assemble in water to form spherical, hollow nanocapsules with diameters of approximately 30-50 nm, i.e., artificial viral capsids. In these nanocapsules, the N-terminus of the β-annulus peptide faces the interior of the hollow capsule, and the C-terminus faces the exterior of the hollow capsule. It has been demonstrated that various substances can be encapsulated in these artificial viral capsids (either in the form of free molecules or in a form that binds to or interacts with the N-terminus of the β-annulus peptide). These encapsulated substances are also called guests, and depending on the type of guests, the diameter of the artificial viral capsid may be somewhat enlarged. It has also been demonstrated that by linking molecules such as gold nanoparticles, 20-nucleotide single-stranded DNA, coiled-coil-forming peptides, human serum albumin, and ribonuclease S near the C-terminus of a β-annulus peptide, artificial viral capsids can be formed whose outer surface is modified with these molecules. It has also been reported that modified artificial viral capsids obtained by linking a nucleic acid aptamer having a length of 40 nucleotides or more to the C-terminus of a β-annulus peptide and then allowing it to self-assemble can be efficiently and specifically delivered (e.g., introduced into specific target cells) via the aptamer moiety (Patent Document 1).

On the other hand, compounds having a polyfluoro structure are known to be stable and low in toxicity in vivo, and to be excellent in uptake into cells and escape from endosomes. Utilizing these properties, studies have been conducted to introduce a polyfluoro structure into oligonucleotides or peptide nucleic acids as a moiety capable of penetrating cell membranes (Patent Documents 2 and 3).

In addition, introduction of polyfluoro structures into peptides has been carried out. For example, it has been reported that fluoropeptides whose constituent residues are fluorine-containing amino acids with polyfluoroalkyl groups introduced into their side chains have good cell membrane permeability (Patent Document 4).

Citation List

Patent Documents

Patent Document 1: Japanese Unexamined Patent Application, First Publication No. 2022-173834 A
Patent Document 2: PCT International Publication No. WO 2012/130941
Patent Document 3: PCT International Publication No. WO 2021/060506
Patent Document 4: PCT International Publication No. WO 2023/048236

Non-Patent Documents

Non-Patent Document 1: Chem. Commun., 2018, vol.54, p.8944.
Non-Patent Document 2: Bioconjugate Chem. 2019, vol.30, p.1636-1641.
Non-Patent Document 3: J. Org. Chem. 2020, vol.85, p.1668-1673.
Non-Patent Document 4: J Pept Sci. 2017, vol.23 (7-8), p.636-643.
Non-Patent Document 5: Kostrzewa-Nowak et al., British Journal of Cancer, 2005, vol.93, p.89-97.

SUMMARY OF INVENTION

Technical Problem

An object of the present invention is to provide an artificial viral capsid that has excellent cell membrane permeability.

Solution to Problem

The present inventors discovered that by modifying the β-annulus peptide of tomato bushy stunt virus (TBSV) with a peptide containing an amino acid residue having a fluorine atom introduced into the side chain, the cell membrane permeability of the artificial viral capsid obtained by self-assembly of the β-annulus peptide can be improved, and thus completed the present invention.

That is, the present invention is as follows.

[1] An artificial viral capsid formed by self-assembly of multiple subunits, wherein
the subunit comprises:

a β-annulus peptide of tomato bushy stunt virus,
a group derived from a fluorine-containing compound, and
a divalent linking group that links the β-annulus peptide to the group derived from a fluorine-containing compound; and
the divalent linking group is linked to a C-terminus of the β-annulus peptide.

[2] The artificial viral capsid according to [1], wherein the fluorine-containing compound is a fluoropeptide.
[3] The artificial viral capsid according to [2], wherein at least one side chain of amino acid residues constituting the fluoropeptide is represented by the following general formula (1):

[Chemical formula 1]

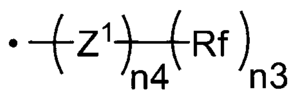

(1)

[wherein $Z^1$ is a linking group other than a di-, tri-, or tetra-valent alkylene group; Rf is a $C_{1-30}$ alkyl group substituted with at least two fluorine atoms (when the $C_{1-30}$ alkyl group has two or more carbon atoms, it may have 1 to 5 ether-bonding oxygen atoms between the carbon atoms), $-SF_3$, or-$SF_4$-$CR^{101}R^{102}$-$CR^{103}R^{104}Cl$ ($R^{101}$, $R^{102}$, $R^{103}$, and $R^{104}$ are each independently a hydrogen atom, a fluorine atom, or a chlorine atom, and two or more of $R^{101}$, $R^{102}$, $R^{103}$, and $R^{104}$ are fluorine atoms); n3 is 1, 2, or 3; n4 is 0 or 1; and a black dot represents a binding site.]
[4] The artificial viral capsid according to [3], wherein Rf is represented by the following general formula (f-1) or (f-2):

[Chemical formula 2]

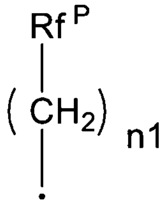

(f-1)

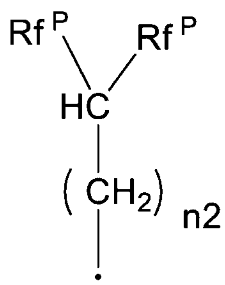

(f-2)

[In the formula, $Rf^P$ represents a fully halogenated $C_{1-10}$ alkyl group containing at least two fluorine atoms (when the fully halogenated $C_{1-10}$ alkyl group has two or more carbon atoms, it may have an ether-bonding oxygen atom between the carbon atoms), n1 represents an integer of 0 to 10, n2 represents an integer of 0 to 9, and a black dot represents a binding site.]

[5] The artificial viral capsid according to [3] or [4], wherein the amino acid residue having a group represented by general formula (1) as a side chain is an amino acid residue having 1 to 3 Rfs linked directly or indirectly to a side chain of a natural amino acid.

[6] The artificial viral capsid according to any one of [1] to [5], wherein the divalent linking group is a bis-maleimide group containing maleimide groups at both ends.

[7] The artificial viral capsid according to any one of [1] to [6], wherein the β-annulus peptide has a cysteine residue at or near the C-terminus, and the divalent linking group is linked to a thiol group derived from the cysteine residue.

[8] A pharmaceutical composition comprising the artificial viral capsid according to any one of [1] to [7].

[9] A drug delivery carrier composition comprising the artificial viral capsid according to any one of [1] to [7].

Advantageous Effects of Invention]

The artificial viral capsid according to the present invention is modified with a group derived from a fluorine-containing compound, and therefore has excellent cell membrane permeability. Therefore, the artificial viral capsid is expected to be used in the pharmaceutical field as a carrier for delivering physiologically active substances or drugs (DDS carrier).

BRIEF DESCRIPTION OF DRAWINGS

FIG. 1 shows the reversed-phase HPLC chart for the purification of a β-annulus-Cys peptide (FIG. 1A) and the MALDI-TOF-MS results (FIG. 1B) in Example 1.

FIG. 2 shows the reversed-phase HPLC chart for the purification of a TMR-β-annulus-Cys peptide (FIG. 2A) and the MALDI-TOF-MS results (FIG. 2B) in Example 1.

FIG. 3 shows the reversed-phase HPLC chart for the purification of a β-annulus-$PEG_2$-maleimide (FIG. 3A) and the MALDI-TOF-MS results (FIG. 3B) in Example 1.

FIG. 4 shows the reversed-phase HPLC chart for the purification of a TMR-β-annulus-$PEG_2$-maleimide (FIG. 4A) and the MALDI-TOF-MS results (FIG. 4B) in Example 1.

FIG. 5 shows the reversed-phase HPLC chart for the purification of a β-annulus-$CAD^{C8F17}$ F peptide (FIG. 5A) and the MALDI-TOF-MS results (FIG. 5B) in Example 1.

FIG. 6 shows the reverse-phase HPLC chart for the purification of a TMR-β-annulus-$CAD^{C8F17}$ F peptide (FIG. 6A) and the MALDI-TOF-MS results (FIG. 6B) in Example 1.

FIG. 7 shows the reversed-phase HPLC chart for the purification of a TMR-$CAD^{C8F17}$ F peptide (FIG. 7A) and the MALDI-TOF-MS results (FIG. 7B) in Example 1.

FIG. 8 shows the measurement results for the particle size distribution (number-based distribution) of a fluoropeptide-modified capsid in Example 1. FIG. 8A shows the results for the fluoropeptide-modified capsid formed in a solution with a β-annulus-$CAD^{C8F17}$ F peptide concentration of 50 μM, and FIG. 8B shows the results for the fluoropeptide-modified capsid formed in a solution with a β-annulus-$CAD^{C8F17}$ F peptide concentration of 10 μM.

FIG. 9 shows TEM images of the capsid modified with a fluoropeptide in Example 1.

FIG. 10 shows the measurement results for the particle size distribution (number-based distribution) of a TMR-labeled fluoropeptide-modified capsid in Example 1.

FIG. 11 shows TMR fluorescence images, merged images of TMR fluorescence images and Hoechst 33342 fluorescence images, and transmitted light images of cells introduced with a TMR-labeled fluoropeptide-modified capsid (top row), cells introduced with a TMR-labeled fluoropeptide (middle row), and cells introduced with a TMR-labeled capsid (bottom row) in Example 1.

FIG. 12 shows the results of measuring the TMR fluorescence intensity in the cells introduced with a TMR-labeled fluoropeptide-modified capsid (left column), the cells introduced with a TMR-labeled fluoropeptide (center column), and the cells introduced with a TMR-labeled capsid (right column) in Example 1.

FIG. 13 shows the results of flow cytometry analysis (left: dot plot, right: histogram) of the untreated cells (A), the cells introduced with a TMR-labeled fluoropeptide-modified capsid (B), the cells introduced with a TMR-labeled fluoropeptide (C), and the cells introduced with a TMR-labeled capsid (D), in Example 1.

FIG. 14 shows the measurement results of the percentage (%) of cells that have taken up TMR (FIG. 14A) and the measurement results of the median TMR fluorescence intensity (FIG. 14B) in Example 1 for the untreated cells, the

cells introduced with a TMR-labeled fluoropeptide-modified capsid, the cells introduced with a TMR-labeled fluoropeptide, and the cells introduced with a TMR-labeled capsid.

FIG. 15 shows the results of measuring the particle size distribution (number-based distribution) of a Dox-encapsulated fluoropeptide-modified capsid in Example 2.

FIG. 16 shows a TEM image of the Dox-encapsulated fluoropeptide-modified capsid in Example 2.

FIG. 17 shows the results of measuring the UV-vis spectra and Dox binding rate (%) of the Dox-encapsulated fluoropeptide-modified capsid and a Dox-encapsulated fluoropeptide-unmodified capsid in Example 2.

FIG. 18 shows the results of measuring the cell viability (%) of the cells introduced with a Dox-encapsulated fluoropeptide-modified capsid in Example 2.

FIG. 19 shows TMR fluorescence images of the cells cultured in a medium containing only a TMR-labeled fluoropeptide-modified capsid (Control), the cells cultured in a medium containing a TMR-labeled fluoropeptide and EIPA (80 μM EIPA), the cells cultured in a medium containing a TMR-labeled fluoropeptide and Pitstop 2 (20 μM Pitstop 2), and the cells cultured in a medium containing a TMR-labeled capsid and Genistein (160 μM Genistein) in Example 3.

FIG. 20 shows the results of measuring the TMR fluorescence intensity of the cells cultured in a medium containing only a TMR-labeled fluoropeptide-modified capsid (Control), the cells cultured in a medium containing a TMR-labeled fluoropeptide and EIPA (EIPA), the cells cultured in a medium containing a TMR-labeled fluoropeptide and Pitstop 2 (Pitstop 2), and the cells cultured in a medium containing a TMR-labeled capsid and Genistein (Genistein) in Example 3.

FIG. 21 shows the reversed-phase HPLC chart (FIG. 21A) and the ESI-MS results (FIG. 21B) in the purification of a β-annulus-CAD$^{C6F13}$ F peptide in Example 4.

FIG. 22 shows the images of the cells introduced with mCherry mRNA using a transfection reagent (left panel), the cells introduced with mCherry mRNA using a CAD$^{C6F13}$ F peptide-modified artificial viral capsid (middle panel), and the cells introduced with mCherry mRNA using an unmodified artificial viral capsid (right panel), in Example 4. The images include Hoechst 33342 fluorescent images, mCherry fluorescent images, merged images of mCherry fluorescent images and Hoechst 33342 fluorescent images, as well as transmitted light images.

FIG. 23 shows the mCherry fluorescence intensity of the cells introduced with mCherry mRNA using a transfection reagent (left column), the cells introduced with mCherry mRNA using a CAD$^{C6F13}$ F peptide-modified artificial viral capsid (middle column), and the cells introduced with mCherry mRNA using an unmodified artificial viral capsid (right column), in Example 4.

DESCRIPTION OF EMBODIMENTS

In the present invention and the present specification, "$C_{p1-p2}$" (p1 and p2 are positive integers satisfying p1<p2) means a group having from p1 to p2 carbon atoms.

In the present invention and the present specification, the "$C_{1-30}$ alkyl group" is an alkyl group having 1 to 30 carbon atoms, and may be linear or branched. The "$C_{2-30}$ alkyl group" is an alkyl group having 2 to 30 carbon atoms, and may be linear or branched. Examples of the $C_{1-30}$ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a tricosyl group, a tetracosyl group, a pentacosyl group, a hexacosyl group, a heptacosyl group, an octacosyl group, a nonacosyl group, a triacontyl group, and the like.

In the present invention and the present specification, the "$C_{1-10}$ alkyl group" is an alkyl group having 1 to 10 carbon atoms, and may be linear or branched. The "$C_{2-10}$ alkyl group" is an alkyl group having 2 to 10 carbon atoms, and may be linear or branched. Examples of the $C_{1-10}$ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, and the like.

In the present invention and the present specification, the "$C_{1-6}$ alkyl group" is an alkyl group having 1 to 6 carbon atoms, and may be linear or branched. Examples of the $C_{1-6}$ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, and the like.

In the present invention and the present specification, the "$C_{6-14}$ aryl group" is an aromatic hydrocarbon group having 6 to 14 carbon atoms, with a $C_{6-12}$ aryl group being particularly preferred. Examples of the $C_{6-14}$ aryl group include a phenyl group, a naphthyl group, an anthryl group, and a 9-fluorenyl group, with a phenyl group being particularly preferred.

In the present invention and the present specification, the "optionally substituted $C_{6-14}$ aryl group" is a group in

which one or more, preferably 1 to 3, hydrogen atoms bonded to carbon atoms of a $C_{6-14}$ aryl group are substituted with other functional groups. When the $C_{6-14}$ aryl group has two or more substituents, the substituents may be the same or different. Examples of the substituent include a nitro group, a halogen atom (fluorine atom, chlorine atom, bromine atom, or iodine atom), a $C_{1-6}$ alkyl group, a $C_{1-6}$ alkoxy group, a methylenedioxy group ($-O-CH_2-O-$), and the like. Examples of the "optionally substituted $C_{6-14}$ aryl group" include a phenyl group, a naphthyl group, an anthryl group, a 4-nitrophenyl group, a 4-methoxyphenyl group, a 2,4-dimethoxyphenyl group, a 3,4-dimethoxyphenyl group, a 4-methylphenyl group, a 2,6-dimethylphenyl group, a 3-chlorophenyl group, a 1,3-benzodioxol-5-yl group, and the like.

In the present invention and the present specification, the "$C_{6-14}$ aryl-$C_{1-6}$ alkyl group" is a group in which one hydrogen atom bonded to a carbon atom of a $C_{1-6}$ alkyl group is substituted with a $C_{6-14}$ aryl group. Examples of the $C_{6-14}$ aryl group in the $C_{6-14}$ aryl-$C_{1-6}$ alkyl group include a phenyl group, a naphthyl group, an anthryl group, a 9-fluorenyl group, and the like, with a phenyl group or a 9-fluorenyl group being particularly preferred. The $C_{1-4}$ alkyl group is preferable as the $C_{1-6}$ alkyl group in the $C_{6-14}$ aryl-$C_{1-6}$ alkyl group. Examples of the $C_{6-14}$ aryl-$C_{1-6}$ alkyl group include a benzyl group, a diphenylmethyl group, a triphenylmethyl group, a 2-phenylethyl group, a 9-anthrylmethyl group, a 9-fluorenylmethyl group, and the like.

In the present invention and the present specification, the term "halogen atom" refers to a fluorine atom, a chlorine atom, a bromine atom, or an iodine atom. The term "halogen atom other than a fluorine atom" refers to a chlorine atom, a bromine atom, or an iodine atom. Preferred examples of the "halogen atom other than a fluorine atom" include a chlorine atom or a bromine atom, with a chlorine atom being particularly preferred.

In the present invention and the present specification, the term "$C_{1-6}$ alkoxy group" refers to a group in which an oxygen atom is bonded to the bonding terminal of a $C_{1-6}$ alkyl group having 1 to 6 carbon atoms. The $C_{1-6}$ alkoxy group may be linear or branched. Examples of the $C_{1-6}$ alkoxy group include a methoxy group, an ethoxy group, a propoxy group, a butoxy group, a tert-butoxy group, a pentyloxy group, a hexyloxy group, and the like.

In the present invention and the present specification, the term "ether-bonding oxygen atom" refers to an oxygen atom that links carbon atoms, and does not include oxygen atoms in which oxygen atoms are linked in series. An alkyl group having Nc carbon atoms (Nc is an integer of two or more) may have a maximum of Nc-1 ether-bonding oxygen atoms.

In the following description, "compound n" means a compound represented by formula (n).

The artificial viral capsid according to the present invention is an artificial viral capsid formed by the self-assembly of multiple subunits, each of which contains a β-annular peptide of TBSV, a group derived from a fluorine-containing compound, and a divalent linking group that links the β-annular peptide to the group derived from a fluorine-containing compound. In the artificial viral capsid, the divalent linking group is linked to the C-terminus of the β-annular peptide, and therefore the group derived from a fluorine-containing compound appears on the outside of the hollow capsule of the artificial viral capsid.

<β-annulus peptide>

The β-annulus peptide of TBSV (hereinafter simply referred to as "β-annulus peptide") is publicly known and is reviewed in Non-Patent Document 1. The β-annulus peptide consists of the amino acid sequence represented by INHVGGTGGAIMAPVAVTRQLVGS (SEQ ID NO: 1). Among the amino acid sequence of SEQ ID NO: 1, the region 1 to 5, the region 10 to 13, and the region 17 to 22 are essential parts for the formation of the artificial viral capsid by self-assembly (hereinafter sometimes referred to as the "β-annulus peptide consensus sequence"), and are the minimum unit capable of forming the artificial viral capsid by self-assembly in water.

The β-annulus peptide used in the present invention and the present specification is not particularly limited, as long as it contains the β-annulus peptide consensus sequence and can form an artificial viral capsid by self-assembly in water. For example, it may be a peptide consisting only of the amino acid sequence represented by SEQ ID NO: 1. The regions of the amino acid sequence represented by SEQ ID NO: 1 other than the β-annulus peptide consensus sequence may be substituted with other amino acid residues, deleted, or added with other amino acid residues or amino acid sequences, as long as they do not inhibit the formation of the artificial viral capsid. Furthermore, an amino acid sequence may be added to the C-terminus and/or N-terminus of SEQ ID NO: 1.

Previous studies have demonstrated that an artificial viral capsid can be formed even when an additional amino acid sequence is added to the C-terminus and/or N-terminus of SEQ ID NO: 1. In the present invention and the present specification, the β-annulus peptide may be a peptide composed of an amino acid sequence in which, for example, 1 to 20 amino acid residues, 1 to 10 amino acid residues, or 1 to 5 amino acid residues are added to the N-terminus and/or C-terminus of the amino acid sequence represented by SEQ ID NO: 1.

The amino acid sequence added to the amino acid sequence represented by SEQ ID NO: 1 is not particularly limited as long as it does not inhibit the formation of an artificial viral capsid by self-assembly, and may be, for example, a linker sequence for linking the β-annulus peptide to another substance that modifies the β-annulus peptide. It is within the skill of those skilled in the art to synthesize a peptide so that a functional group useful for linker binding or the linker itself is

present at the N-terminus and/or C-terminus of the amino acid sequence represented by SEQ ID NO: 1.

In the present invention and the present specification, the term "subunit" refers to a unit molecule that forms an artificial viral capsid through self-assembly. Each subunit contains at least a β-annulus peptide. However, the multiple subunits that self-assemble into a single artificial viral capsid are not necessarily identical to one another; for example, embodiments in which subunits modified with a fluorine-containing compound and subunits without a fluorine-containing compound are mixed are also contemplated. Artificial viral capsids formed by the self-assembly of multiple subunits, each of which is modified with a different fluorine-containing compound, are also contemplated. As described in Non-Patent Documents 1 to 4, self-assembly refers to the spontaneous combination of multiple β-annulus peptide-containing subunits in water or an aqueous solution to form a roughly spherical artificial viral capsid, or the structure thus formed. The diameter of each artificial viral capsid formed by self-assembly (as measured by dynamic light scattering, DLS) may vary depending on the presence and type of guest, but is typically 30 to 100 nm. A single artificial viral capsid is estimated to contain approximately 60 subunits. The pH of the aqueous solution during self-assembly is typically 5 to 9, and can be, for example, 6 to 8, or 6.5 to 7.5. The concentration of the subunits during self-assembly can be, for example, 1 to 50 μM, 3 to 40 μM, or 5 to 30 μM.

The β-annulus peptides used in the present invention and the present specification preferably have a cysteine residue at or near the C-terminus, specifically, 1 to 5 amino acid residues from the C-terminus, and more preferably 2 to 5 amino acid residues from the C-terminus. Note that the term "1 amino acid residue from the C-terminus" refers to the amino acid residue at the C-terminus. Examples of β-annulus peptides having a cysteine residue at or near the C-terminus include a peptide composed of an amino acid sequence (SEQ ID NO: 2) in which the second amino acid residue (G) from the C-terminus of SEQ ID NO: 1 is substituted with a cysteine residue, a peptide (SEQ ID NO: 3) in which the C-terminal amino acid residue (S) of SEQ ID NO: 1 is substituted with a cysteine residue, and a peptide composed of an amino acid sequence obtained by adding a peptide composed of 1 to 4 amino acids to the C-terminus of these amino acid sequences. Furthermore, the peptide may also be an amino acid sequence obtained by adding an amino acid sequence composed of 1 to 5 amino acids containing a cysteine residue to the C-terminus of the amino acid sequence of SEQ ID NO: 1. The amino acid sequence having a cysteine residue to be added to the C-terminus of the amino acid sequence of SEQ ID NO: 1 is preferably an amino acid sequence of 1 to 5 amino acids in which all residues other than the cysteine residue are glycine residues, since this minimizes the effect on artificial viral capsid formation, and more preferably an amino acid sequence of 2 to 5 amino acids in which the second residue from the C-terminus is a cysteine residue and the remaining residues are all glycine residues.

The β-annulus peptide used in the present invention and the present specification may have a cysteine residue at or near the N-terminus, specifically 1 to 5 amino acid residues from the N-terminus, and preferably one amino acid residue from the N-terminus. Having a cysteine residue at or near the N-terminus makes it easier to modify the N-terminus of the β-annulus peptide with other substances. Examples of the β-annulus peptide having a cysteine residue at or near the N-terminus include a peptide composed of an amino acid sequence in which an amino acid sequence composed of 1 to 5 amino acids containing a cysteine residue is added to the N-terminus of the amino acid sequence of SEQ ID NO: 1. The amino acid sequence having a cysteine residue added to the N-terminus of the amino acid sequence of SEQ ID NO: 1 is preferably an amino acid sequence of 1 to 5 amino acids in which all residues except the cysteine residue are glycine residues, since this minimizes the effect on artificial viral capsid formation. An amino acid sequence of 2 to 5 amino acids in which the first residue from the N-terminus is a cysteine residue and the remaining residues are all glycine residues is more preferable.

<Group derived from a fluorine-containing compound>

The term "group derived from a fluorine-containing compound" used in the present invention and the present specification refers to a group in which one hydrogen atom is removed from a fluorine-containing compound having at least one fluorine atom to form a binding site. In order to obtain a more sufficient effect of improving cell membrane permeability due to fluorine, the "group derived from a fluorine-containing compound" used in the present invention is preferably a group having a group represented by the following general formula (1). In general formula (1), a black dot represents a binding site.

[Chemical formula 3]

$$\bullet - \left( Z^1 \right)_{n4} \left( Rf \right)_{n3} \qquad (1)$$

In general formula (1), Rf is a $C_{1-30}$ alkyl group substituted with at least two fluorine atoms, $-SF_5$, or

$-SF_4-CR^{101}R^{102}-CR^{103}R^{104}Cl$.

When Rf is a $C_{1-30}$ alkyl group substituted with at least two fluorine atoms, the $C_{1-30}$ alkyl group, when it has two or more carbon atoms (in the case of a $C_{2-30}$ alkyl group), may have 1 to 5 ether-bonding oxygen atoms between the carbon atoms.

In Rf, one or more hydrogen atoms bonded to a carbon atom may be further substituted with a halogen atom other than a fluorine atom. Here, the $C_{1-30}$ alkyl group for Rf is preferably a $C_{1-20}$ alkyl group, more preferably a $C_{1-10}$ alkyl group, even more preferably a $C_{2-10}$ alkyl group, and still even more preferably a $C_{2-8}$ alkyl group. When the $C_{1-30}$ alkyl group is a $C_{2-30}$ alkyl group, it may have 1 to 5 ether-bonding oxygen atoms between the carbon atoms. In Rf, the number of hydrogen atoms substituted with fluorine atoms is not particularly limited as long as it is two or more, and is, for example, preferably three or more, more preferably 6 or more, and even more preferably 7 or more.

Examples of Rf include a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, a perfluorodecyl group, a difluoromethyl group, a 1,1-difluoroethyl group, a 2,2-difluoroethyl group, a 1,1,2,2-tetrafluoroethyl group, a 1,1,2,2,3,3-hexafluoropropyl group, a 1,1,2,3,3,3-hexafluoropropyl group, a 1,1,2,2,3,3-hexafluorohexyl group, a 1,1,2,2,3,3-hexafluorooctyl group, a 1,1,2,2,3,3-hexafluorodecyl group, a 1,1,2,2,3,3-hexafluorooctadecyl group, a 1,1,2,2,3,3-hexafluorohexacosyl group, and the like.

When Rf is a group having two carbon atoms, Rf is preferably a group in which at least four or more hydrogen atoms bonded to carbon atoms are substituted with fluorine atoms, such as a pentafluoroethyl group, rather than a 1,1,1-trifluoroethyl group ($CF_3-CH_2-$). When Rf is a group having three carbon atoms, Rf is preferably a linear group, and in the case of a branched group, a 1,1,1,3,3,3-hexafluoropropan-2-yl group ($(CF_3)_2-CH-$) or $(CF_3)_2-CF-$group is preferable. When Rf is a group having four carbon atoms, Rf may be a linear group or a branched group. In the case of a branched group, Rf is preferably a group in which hydrogen atoms bonded to carbon atoms constituting the alkylene group moiety are substituted with fluorine atoms, or a fully fluorinated group.

Rf is preferably a group represented by the following general formula (f-1) or (f-2). In general formulas (f-1) and (f-2), a black dot represents a binding site.

[Chemical formula 4]

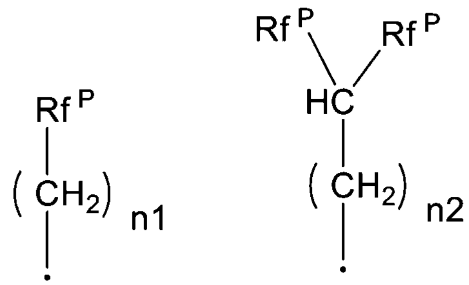

(f-1) (f-2)

In general formulas (f-1) and (f-2), $Rf^P$ represents a fully halogenated $C_{1-10}$ alkyl group containing at least two fluorine atoms. $Rf^P$ is a $C_{1-10}$ alkyl group in which all of the hydrogen atoms are substituted with halogen atoms, and at least two of these halogen atoms are fluorine atoms. When $Rf^P$ has two or more carbon atoms, that is, when it is a fully halogenated $C_{2-10}$ alkyl group, it may have 1 to 5 ether-bonding oxygen atoms between the carbon atoms. In general formula (f-2), the two $Rf^P$s may be the same or different groups.

In the following general formula (f-1) or (f-2), n1 is an integer of 0 to 10, and n2 is an integer of 0 to 9. When n1 and n2 are 0, they both represent a single bond. That is, when n1 is 0, the group represented by general formula (f-1) is $Rf^P-$, and when n2 is 0, the group represented by general formula (f-2) is $(Rf^P)_2-CH-$.

When Rf is a group represented by general formula (f-1), Rf is preferably a group in which $Rf^P$ is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n1 is an integer of 0 to 4; more preferably a group in which $Rf^P$ is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n1 is an integer of 0 to 2; even more preferably a group in which $Rf^P$ is a trifluoromethyl group, a pentafluoroethyl group, a heptafluoropropyl group, a

nonafluorobutyl group, a perfluoropentyl group, or a perfluorohexyl group, and n1 is an integer of 0 to 2 (excluding groups in which n1 is 1 and $Rf^P$ is a trifluoromethyl group).

When Rf is a group represented by general formula (f-2), Rf is preferably a group in which $Rf^P$ is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n2 is an integer of 0 to 4; more preferably a group in which $Rf^P$ is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, or a perfluorodecyl group, and n2 is an integer of 0 to 2; even more preferably a group in which $Rf^P$ is a trifluoromethyl group, a pentafluoroethyl group, a heptafluoropropyl group, a nonafluorobutyl group, a perfluoropentyl group, or a perfluorohexyl group, and n2 is an integer of 0 to 2 (excluding groups in which n2 is 0 or 1 and $Rf^P$ is a trifluoromethyl group).

Examples of Rf include a difluoromethyl group, a 1,1-difluoroethyl group, a 2,2-difluoroethyl group, a 1,1,2,2-tetrafluoroethyl group, a 1,1,2,2,3,3-hexafluoropropyl group, a 1,1,2,3,3,3-hexafluoropropyl group, and the like.

When Rf is a group represented by $-SF_4-CR^{101}R^{102}-CR^{103}R^{104}Cl$, $R^{101}$, $R^{102}$, $R^{103}$, and $R^{104}$ are each independently a hydrogen atom, a fluorine atom, or a chlorine atom, provided that two or more of $R^{101}$, $R^{102}$, $R^{103}$, and $R^{104}$ are fluorine atoms. Specific examples of the group represented by $-SF_4-CR^{101}R^{102}-CR^{103}R^{104}Cl$ include $-SF_4-CF_2-CF_2Cl$, $-SF_4-CF_2-CFCl_2$, $-SF_4-CF_2-CHF-Cl$, -SF4-$CF_2-CCl_3$, $-SF_4-CF_2-CHCl_2$, $-SF_4-CF_2-CH_2Cl$, $-SF_4-CFCl-CFCl_2$, $-SF_4-CFCl-CHF-Cl$, and $-SF_4-CHF-CHF-Cl$.

In general formula (1), $Z^1$ represents a di-, tri-, or tetra-valent linking group other than an alkylene group, n3 represents 1, 2, or 3, and n4 represents 0 or 1. When n4 is 0, - $(Z^1)$n4- represents a single bond. $Z^1$ is not particularly limited as long as it is a di-, tri-, or tetra-valent group other than an alkylene group. For example, $Z^1$ can be an alkylene group, an oxygen atom (-O-), a sulfur atom (-S-), -NH-, $-N(CH_3)-$, $-N(C_2H_5)-$, $-N(C_3H_7)-$, a trivalent nitrogen atom, -C(=O)-, $-S(=O)_2-$, a group obtained by removing 2 to 4 hydrogen atoms from a cycloalkane, a group obtained by removing 2 to 4 hydrogen atoms from an aromatic ring (aryl group), a group obtained by removing 2 to 4 hydrogen atoms from a heterocyclic ring (heteroaryl group), or a combination thereof. The aryl group and the heteroaryl group may be any of the groups listed above, except for groups consisting only of alkylene groups and groups in which the linking moiety to Rf is an alkylene group.

When $Z^1$ is an alkylene group, an oxygen atom (-O-), a sulfur atom (-S-), -NH-, - $N(CH_3)-$, $-N(C_2H_5)-$, $-N(C_3H_7)-$, -C(=O)-, $-S(=O)_2-$, a group obtained by removing two hydrogen atoms from a cycloalkane, a group obtained by removing two hydrogen atoms from an aromatic ring, a group obtained by removing two hydrogen atoms from a heterocyclic ring, or a combination thereof, $Z^1$ is a divalent linking group, and the group represented by general formula (1) is a group having one Rf group. When $Z^1$ has a trivalent nitrogen atom, two Rf groups can be bound to the nitrogen atom directly or via another divalent linking group, thereby making the group represented by general formula (1) a group having two Rf groups.

$Z^1$ may be a linking group having a group (cyclic group) obtained by removing a hydrogen atom from a ring, or may be a linking group without a cyclic group. When $Z^1$ has a cyclic group, the group represented by general formula (1) can be a group having two or three Rf groups. Examples of the cyclic group include a group obtained by removing 2 to 4 hydrogen atoms from a cycloalkane, an aromatic ring, or a heterocyclic ring. As the heterocyclic ring, a ring in which 1 to 3 carbon atoms in an aromatic ring are substituted with one or more atoms selected from the group composed of a nitrogen atom, an oxygen atom, and a sulfur atom, is preferable. Furthermore, the cyclic group may be a group obtained by removing a hydrogen atom from a monocyclic ring, or a group obtained by removing a hydrogen atom from a fused ring. As the cyclic group contained in $Z^1$ in general formula (1), a group obtained by removing 2 to 4 hydrogen atoms from cyclohexane, benzene, imidazole, or indole is preferable. For example, when $Z^1$ is a linking group having a cyclic group formed by removing 2 to 4 hydrogen atoms from benzene, the cyclic group is a 1,4-phenylene group, a 1,3-phenylene group, a 1,5-phenylene group, or a 1,3,5-substituted phenyl group, and a 1,4-phenylene group or a 1,3,5-substituted phenyl group is preferable.

Specifically, examples of $Z^1$ in general formula (1) include -C(=O)-, -C(=O)-O-, -O-C(=O)-, -NH-C(=O)-O-, -O-C(=O)-NH-, -C(=O)-NH-, -NH-C(=O)-, -S-S-, $-S(=O)_2-NH-$, $-NH-S(=O)_2-$, $-S(=O)_2-NH-S(=O)_2-$, and -C(=O)-NH-Ph- (wherein -Ph- is a 1,4-phenylene group, a 1,3-phenylene group, a 1,5-phenylene group, or a 1,3,5-substituted phenyl group). Furthermore, linking groups formed by combining any of these groups with a $C_{1-6}$ alkylene group are also preferable. $Z^1$ in general formula (1) is preferably one in which the linking moiety to Rf is an oxygen atom.

$Z^1$ in general formula (1) is preferably a linking group represented by the following general formula (2). In general formula (2), a black dot represents a binding site.

[Chemical formula 5]

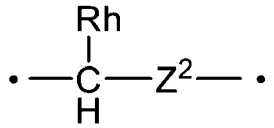

(2)

In general formula (2), $Z^2$ is a di-, tri-, or tetra-valent linking group other than an alkylene group. $Z^2$ is not particularly limited as long as it is a di-, tri-, or tetra-valent group other than an alkylene group. For example, $Z^2$ can be an alkylene group, an oxygen atom (-O-), a sulfur atom (-S-), -NH-, $-N(CH_3)-$, $-N(C_2H_5)-$, $-N(C_3H_7)-$, a trivalent nitrogen atom, -C(=O)-, $-S(=O)_2-$, a group obtained by removing 2 to 4 hydrogen atoms from a cycloalkane, a group obtained by removing 2 to 4 hydrogen atoms from an aromatic ring (aryl group), a group obtained by removing 2 to 4 hydrogen atoms from a heterocyclic ring (heteroaryl group), or a combination thereof. The aryl group and the heteroaryl group may be any of the groups listed above, except for the groups consisting only of alkylene groups and groups in which the linking moiety to Rf is an alkylene group.

As $Z^2$ in general formula (2), specifically, the same groups as those exemplified for $Z^1$ can be used. $Z^2$ in general formula (2) is preferably -C(=O)-, -C(=O)-O-, -O-C(=O)-, -NH-C(=O)-O-, -O-C(=O)-NH-, -C(=O)-NH-, -NH-C(=O)-, -S-S-, $-S(=O)_2-NH-$, $-NH-S(=O)_2-$, $-S(=O)_2-NH-S(=O)_2-$, or -C(=O)-NH-Ph- (wherein -Ph- is a 1,4-phenylene group, a 1,3-phenylene group, a 1,5-phenylene group, or a 1,3,5-substituted phenyl group).

In general formula (2), Rh is a hydrogen atom or a $C_{1-6}$ alkyl group. When Rh is a $C_{1-6}$ alkyl group, Rh is preferably a $C_{1-3}$ alkyl group, more preferably a methyl group or an ethyl group.

Examples of the group represented by general formula (1) include a group in which $Z^1$ is a group represented by general formula (2) and Rf is a group represented by general formula (f-1) or (f-2). Among these, groups in which $Z^2$ in general formula (2) is-C(=O)-, -C(=O)-O-, -O-C(=O)-, -NH-C(=O)-O-, -O-C(=O)-NH-, -C(=O)-NH-, -NH-C(=O)-, -S-S-, $-S(=O)_2-NH-$, $-NH-S(=O)_2-$, $-S(=O)_2-NH-S(=O)_2-$, -C(=O)-NH-Ph- (-Ph- is a 1,4-phenylene group, a 1,3-phenylene group, a 1,5-phenylene group, or a 1,3,5-substituted phenyl group), Rh is a hydrogen atom or a $C_{1-6}$ alkyl group, and Rf is a group represented by general formula (f-1) or (f-2), are preferable.

The group derived from a fluorine-containing compound in the subunit of the artificial viral capsid according to the present invention is not particularly limited, as long as it is a group having at least one fluorine atom and does not inhibit the formation of an artificial viral capsid by self-assembly of the β-annulus peptide when linked to the β-annulus peptide. Examples of the fluorine-containing compound having the group include groups derived from the compounds in which a fluorine atom or a fluorine-containing group is introduced into a peptide, nucleic acid, low-molecular-weight compound, or a chimeric molecule composed of two or more of these. It is preferable that at least one hydrogen atom in the peptide, nucleic acid, or low-molecular-weight compound is replaced by the group represented by general formula (1).

The group derived from a fluorine-containing compound possessed by the artificial viral capsid according to the present invention is preferably a fluorine-containing amino acid residue or a fluoropeptide. The fluorine-containing amino acid is preferably an amino acid having a group represented by general formula (1) bound to its α-carbon. The fluoropeptide is preferably a peptide in which at least one side chain of an amino acid residue constituting the peptide is a group represented by general formula (1) or a group containing the group.

The fluoropeptide contained in the artificial viral capsid according to the present invention is preferably an amino acid residue whose side chain is a group represented by general formula (1) [-($Z^1$)n4-(Rf)n3], or a peptide having one or more amino acid residues in which 1 to 3 Rf groups are linked directly or indirectly to the side chain of a natural amino acid. Specifically, the fluoropeptide contained in the artificial viral capsid according to the present invention is preferably a peptide having at least one amino acid residue in which one or two hydrogen atoms of the amino group in the side chain of an arginine, asparagine, glutamine, or lysine residue are substituted with -Rf or -$Z^3$-(Rf)n5 ($Z^3$ is a linking group other than a di-, tri-, or tetra-valent alkylene group, excluding groups in which the linking moiety to Rf is an alkylene group; n5 is 2, 3, or 4); a peptide having at least one amino acid residue in which the hydrogen atom of the imino group in the side chain of an arginine residue is substituted with -Rf or -$Z^3$-(Rf)n5; a peptide having at least one amino acid residue in which the hydrogen atom of the carboxyl group in the side chain of an aspartic acid or glutamic acid residue is substituted with -Rf or -$Z^3$-(Rf)n5; a peptide having at least one amino acid residue in which the hydrogen atom of the thiol group in the side chain of a cysteine or methionine residue is substituted with -Rf or -$Z^3$-(Rf)n5; a peptide having at least one amino acid residue in which the hydrogen atom of a hydroxyl group in the side chain of a serine, threonine, or tryptophan residue is substituted with -Rf or -$Z^3$-(Rf)n5; a peptide having at least one amino acid residue in which 1 to 3 hydrogen atoms on the benzene ring in the side chain of a tyrosine or phenylalanine residue are substituted with -Rf or -$Z^3$-(Rf)n5; a peptide having at least one amino acid residue in which 1 to 3 hydrogen atoms on the imidazole ring in the side chain of a histidine residue are substituted with -Rf or -$Z^3$-(Rf)n5; or a peptide having at least one amino acid residue in which 1 to 3 hydrogen atoms on the indole ring in the side chain of a tryptophan residue are substituted with -Rf or -$Z^3$-(Rf)n5.

$Z^3$ is not particularly limited as long as it is a divalent to tetravalent group other than an alkylene group. For example, $Z^3$ can be an alkylene group, an oxygen atom (-O-), a sulfur atom (-S-), -NH-, $-N(CH_3)-$, $-N(C_2H_5)-$, $-N(C_3H_7)-$, a trivalent nitrogen atom, - C(=O)-, $-S(=O)_2-$, a group in which 2 to 4 hydrogen atoms are removed from a cycloalkane, a group in which 2 to 4 hydrogen atoms are removed from an aromatic ring (aryl group), a group in which 2 to 4 hydrogen atoms are removed from a heterocycle (heteroaryl group), or a combination thereof. Examples of the aryl group and the heteroaryl group include the groups listed above. However, these groups exclude groups consisting solely of alkylene groups and groups in which the linking moiety to Rf is an alkylene group. Specifically, the same linking groups as those for $Z^1$ and $Z^2$ can be used for $Z^3$.

Substitution of a hydrogen atom of an amino group, an imino group, a carboxy group, a hydroxy group, a thiol group, a benzene ring, an imidazole ring, an indole ring, or the like in the side chain of a natural amino acid with -Rf or $-Z^3$-(Rf)n5 can be carried out by a general synthetic reaction such as an ester reaction.

Examples of the fluoropeptide contained in the artificial viral capsid according to the present invention include a peptide having at least one amino acid residue having - ($Z^1$)n4-(Rf)n3 in its side chain. Among the amino acid residues constituting the peptide, at least one amino acid residue may have -($Z^1$)n4-(Rf)n3 in its side chain, and all of the amino acid residues may also have -($Z^1$)n4-(Rf)n3 in their side chains. When a single peptide molecule contains two or more amino acid residues having -($Z^1$)n4-(Rf)n3 in their side chains, these multiple -($Z^1$) n4-(Rf)n3 may be the same or different. Furthermore, within the peptide, the amino acid residue having a side chain containing -($Z^1$)n4-(Rf)n3 may be located at the N-terminus, C-terminus, or anywhere other than the terminal.

The fluoropeptide contained in the artificial viral capsid according to the present invention may be a peptide composed of two or more amino acids, and a peptide composed of three or more amino acids is also preferable. The number of amino acid residues constituting the fluoropeptide contained in the artificial viral capsid according to the present invention is preferably 2 to 20, more preferably 3 to 20, even more preferably 3 to 10, and still even more preferably 3 to 6.

Among the fluoropeptides contained in the artificial viral capsid according to the present invention, amino acid residues whose side chains are not -($Z^1$)n4-(Rf)n3 are not particularly limited, and may be $\alpha$-amino acid residues, $\beta$-amino acid residues, $\gamma$-amino acid residues, or $\delta$-amino acid residues. Furthermore, they may be L-amino acid residues or D-amino acid residues. The amino acid residues whose side chains are not -($Z^{1)}$n4-(Rf)n3 in the fluoropeptides contained in the artificial viral capsid according to the present invention are preferably amino acid residues of protein-constituting amino acids, D-isomers thereof, and modified amino acids in which the side chains of these amino acids are modified.

Examples of the protein-constituting amino acid include glycine, alanine, valine, leucine, isoleucine, serine, threonine, phenylalanine, tyrosine, tryptophan, asparagine, glutamine, proline, aspartic acid, glutamic acid, lysine, arginine, histidine, and the like. Furthermore, examples of the modified protein-constituting amino acid include amino acids in which the hydrogen atom of the amino group in the side chain of lysine, arginine, or histidine is substituted with the amino-protecting groups listed above or a Pbf (N-$\omega$-(2,2,4,6,7-pentamethyldihydrobenzofuran-5-sulfonyl) group; amino acids in which the hydrogen atom of the carboxyl group in the side chain of aspartic acid or glutamic acid is substituted with the carboxy-protecting groups listed above or a tert-butyl alkyl group; and amino acids in which the hydrogen atom of the thiol group in cysteine is substituted with a benzyl group.

The fluoropeptide contained in the artificial viral capsid according to the present invention is preferably a peptide composed of 3 to 20, preferably 3 to 10, more preferably 3 to 6 amino acid residues, in which the end linked to the divalent linking group is a cysteine residue and the second residue from the end opposite the end linked to the divalent linking group is a fluorine-containing amino acid residue. The fluoropeptide contained in the artificial viral capsid according to the present invention is particularly preferably a peptide composed of 3 to 20, preferably 3 to 10, more preferably 3 to 6 amino acid residues, in which the N-terminus is a cysteine residue and the second residue from the C-terminus is an amino acid residue in which a hydrogen atom in the side chain of a natural amino acid is substituted with -Rf or-$Z^{3-(}$Rf)n5; more preferably a peptide composed of 3 to 20, preferably 3 to 10, more preferably 3 to 6 amino acid residues, in which the N-terminus is a cysteine residue and the second residue from the C-terminus is a fluorine-containing amino acid residue in which a hydrogen atom in the side chain of a natural amino acid is substituted with -Rf or-$Z^3$-(Rf)n5, and the amino acid residues second from the N-terminus to third from the C-terminus are, independently, alanine residues or glycine residues; and even more preferably a peptide composed of 3 to 20, preferably 3 to 10, and more preferably 3 to 6 amino acid residues, in which the N-terminus is a cysteine residue, the second residue from the C-terminus is an amino acid residue in which a hydrogen atom in the side chain of a natural amino acid is substituted with -Rf or -$Z^3$-(Rf)n5, the amino acid residues second from the N-terminus to third from the C-terminus are, independently, alanine or glycine residues, and the C-terminus is a phenylalanine residue. The amino acid residue in which a hydrogen atom in the side chain of a natural amino acid is substituted with -Rf or-$Z^3$-(Rf)n5 is preferably any one of the following: an amino acid residue in which one or two hydrogen atoms of the amino group in the side chain of an arginine, asparagine, glutamine, or lysine residue are substituted with -Rf or -$Z^3$-(Rf)n5 ($Z^3$ is a linking group other than a di-, tri-, or tetra-valent alkylene group, excluding groups in which the linking moiety to Rf is an alkylene group; n5 is 2, 3, or 4); an amino acid residue in which the hydrogen atom of the imino group in the side chain of an arginine residue is substituted with -Rf or -$Z^3$-(Rf)n5; an amino acid residue in which the hydrogen atom of the carboxyl group in the side chain of an aspartic acid or glutamic acid residue is substituted with -Rf

or -Z$^3$-(Rf)n5; an amino acid residue in which the hydrogen atom of the thiol group in the side chain of a cysteine or methionine residue is substituted with -Rf or -Z$^3$-(Rf)n5; an amino acid residue in which the hydrogen atom of the hydroxyl group in the side chain of a serine, threonine, or tryptophan residue is substituted with -Rf or -Z$^3$-(Rf)n5; an amino acid residue in which 1 to 3 hydrogen atoms of the benzene ring in the side chain of a tyrosine residue or phenylalanine residue are substituted with -Rf or -Z$^3$-(Rf)n5; an amino acid residue in which 1 to 3 hydrogen atoms on the imidazole ring in the side chain of a histidine residue are substituted with -Rf or - Z$^3$-(Rf)n5; or an amino acid residue in which 1 to 3 hydrogen atoms on the indole ring in the side chain of a tryptophan residue are substituted with -Rf or -Z$^3$-(Rf)n5.

In the artificial viral capsid according to the present invention, when the divalent linking group is linked to the N-terminus of the fluoropeptide, the C-terminus of the fluoropeptide may be protected with a protecting group. The protecting group at the C-terminus is not particularly limited as long as it is a carboxy-protecting group, and, for example, a carboxy-protecting group used in peptide synthesis can be used. Specific examples of the carboxy-protecting group include a protecting group selected from the group represented by the following general formula (p-1), a 2-(9,10-dioxo)anthrylmethyl group, a benzyloxymethyl group, and a phenacyl group. In general formula (p-1), R$^3$ is an optionally substituted C$_{6-14}$ aryl group, and R$^4$ and R$^5$ are each independently a hydrogen atom or an optionally substituted C$_{6-14}$ aryl group. Furthermore, a black dot represents a binding site.

[Chemical formula6]

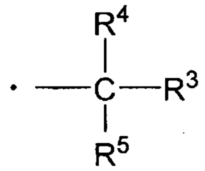

(p-1)

Examples of the carboxy-protecting group include a benzyl, a diphenylmethyl, a triphenylmethyl, a 4-nitrobenzyl, a 4-methoxybenzyl, a 2,4-dimethoxybenzyl, a 3,4-dimethoxybenzyl, a 4-methylbenzyl, a 2,6-dimethylbenzyl, a 3-chlorobenzyl, a 9-anthrylmethyl, a piperonyl, 2-(9,10-dioxo)anthrylmethyl, a benzyloxymethyl, a phenacyl, or the like. The carboxy-protecting group at the C-terminus is preferably a benzyl or a triphenylmethyl group, more preferably a benzyl group, due to its ability to be deprotected under mild conditions.

In the artificial viral capsid according to the present invention, when the divalent linking group is linked to the N-terminus of the fluoropeptide, the N-terminus of the fluoropeptide may be protected with an amino-protecting group. The N-terminal protecting group is not particularly limited as long as it is an amino-protecting group, and, for example, an amino-protecting group used in peptide synthesis can be used. Examples of the amino-protecting group include carbamate-based protecting groups such as a tert-butoxycarbonyl (Boc) group, a 9-fluorenylmethyloxycarbonyl (Fmoc) group, a benzyloxycarbonyl (Cbz) group, an allyloxycarbonyl (Alloc) group, a 2,2,2-trichloroethoxycarbonyl (Troc) group, or the like. A tert-butoxycarbonyl (Boc) group or a 9-fluorenylmethyloxycarbonyl (Fmoc) group is preferable due to its ability to be deprotected under mild conditions.

The fluoropeptide contained in the artificial viral capsid according to the present invention can be produced by a general peptide synthesis method, except that an amino acid having at least one side chain of -(Z$^1$)n4-(Rf)n3 is used as a raw amino acid. For example, it can be produced by a solid-phase peptide synthesis method. The fluoropeptide can be easily synthesized using an automated peptide synthesizer with an amino acid having a side chain of -(Z$^1$)n4-(Rf)n3 as a material. As the fluorine-containing amino acid, an amino acid having a side chain of-(Z$^{1)}$n4-(Rf)n3 is preferable.

Peptides can be synthesized by sequentially condensing amino acids whose C-terminus is bound to a solid support with amino acids whose amino groups are protected, and then cleaving the peptide from the solid support. It is preferable to use amino acid raw materials whose amino groups are protected with a Boc group or an Fmoc group. It is also preferable to use amino acid raw materials whose side chain functional groups are protected with a protecting group. Examples of the protecting group for side chain functional groups include a Boc group, a triphenylmethyl group, a benzyl group, a 2,2,5,7,8-pentamethylchroman-6-sulfonyl (Pmc) group, and the like.

Examples of the condensing agent that forms a peptide bond include an N,N-dicyclohexylcarbodiimide (DCC), a 1-ethyl-3-(3'-dimethylaminopropyl)carbodiimide (WSC), a benzotriazol-1-yloxy-trisdimethylaminophosphonium hexafluorophosphate (BOP), a benzotriazol-1-yloxytrispyrrolizinophosphonium hexafluorophosphate (pyBOP), a 2-(1H-benzotriazol-1-yl)-1,1,3,3-tetramethyluronium hexafluorophosphate (HBTU), a 2-(1H-benzotriazol-1-yl)-1,1,3,3-tetramethyluronium tetrafluoroborate, a 1-cyano-2-ethoxy-2-oxoethylideneaminooxy)dimethylaminomorpholinocarbenium hexafluorophosphate (COMU), and the like. Furthermore, an N-hydroxybenzotriazole (HOBt) and an ethyl (hydroxyimino) cyanoacetate (oxyma) can also be used in a mixture with the aforementioned condensing agents at a preferred ratio.

Peptide bond formation may be achieved by activating the carboxy terminus, and examples of such activators include an N-hydroxysuccinimide, a p-nitrophenyl ester, a pentafluorophenyl ester, and the like. Examples of the base

used for forming a peptide bond include a triethylamine, adiisopropylethylamine (DIPEA), and the like. Examples of the solvent used in peptide bond formation reactions include a chloroform, a dichloromethane (DCM), a dichloroethane (DCE), an acetonitrile (MeCN), an N,N-dimethylformamide (DMF), a dimethyl sulfoxide (DMSO), an N-methyl-2-pyrrolidone (NMP), and the like.

The Boc and Fmoc groups, which are amino-terminal protecting groups for the peptides or amino acids, can be removed with a trifluoroacetic acid (TFA) and a piperidine, respectively. The side-chain protecting groups on the amino acid residues of the peptides can be removed with, for example, TFA, a hydrogen fluoride (HF), a trifluoromethanesulfonic acid, or the like.

In addition, in the solid-phase peptide synthesis method, TFA, for example, can be used as a method for cleaving a peptide with protected side-chain functional groups of its amino acid residues from the solid-phase peptide synthesis resin. The cleavage of the peptide from the solid-phase peptide resin and the removal of the protecting group from the side chain functional group of the amino acid residue can be carried out simultaneously in the same reaction system. Alternatively, they can be carried out independently. As for the solid-phase peptide synthesis resin, commercially available resins such as a 4-hydroxymethyl-3-methoxyphenoxybutyric acid-benzhydrylamine-polystyrene resin, a p-benzyloxybenzyl alcohol-polystyrene resin, an oxime resin, or the like can be used.

The target peptide or its intermediate can be isolated and purified by various methods, such as ion chromatography, gel filtration chromatography, reverse phase chromatography, normal phase chromatography, recrystallization, extraction, fractional crystallization, or the like. Furthermore, the peptide thus obtained can be converted into its respective salt by a conventional method.

The protecting group of the amino group or the carboxy group of the produced fluoropeptide can be deprotected as necessary. The deprotection can be carried out by a conventional method depending on the type of the protecting group.

<Divalent Linking Group>

The divalent linking group linking the β-annulus peptide and the group derived from a fluorine-containing compound is not particularly limited as long as it is a divalent organic group. For example, $Z^1$ can be an alkylene group, an oxygen atom (-O-), a sulfur atom (-S-), -NH-, $-N(CH_3)-$, $-N(C_2H_5)-$, $-N(C_3H_7)-$, a trivalent nitrogen atom, -C(=O)-, $-S(=O)_2-$, a group obtained by removing 2 to 4 hydrogen atoms from a cycloalkane, a group obtained by removing 2 to 4 hydrogen atoms from an aromatic ring (aryl group), a group obtained by removing 2 to 4 hydrogen atoms from a heterocycle (heteroaryl group), or a combination thereof. As the aryl group and the heteroaryl group, the groups mentioned above can be used.

The divalent linking group possessed by the artificial viral capsid according to the present invention is preferably a divalent group having at least one group capable of selectively and easily binding to a thiol group, as it allows for easy binding with a cysteine residue or the like. Examples of the group capable of selectively and easily binding to a thiol group include a maleimide group, an iodoacetyl group, a bromoacetyl group, and the like. Examples of the divalent linking group include bis-maleimide groups containing maleimide groups at both ends, such as the group represented by the following general formula (3):

[Chemical formula 7]

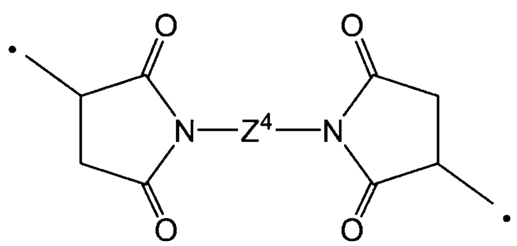

(3)

In general formula (3), a black dot represents a binding site. One of the two black dots binds to a β-annulus peptide, and the other binds to a group derived from a fluorine-containing compound. By utilizing a chemical reaction that forms a thioether group from a maleimide group and a thiol group, easy binding is possible with the thiol group of a cysteine residue in the β-annulus peptide or with the thiol group in a group derived from a fluorine-containing compound. The reaction between the thiol group and the maleimide group can be carried out using a commonly used organic synthesis reaction.

In general formula (3), $Z^4$ represents an alkylene group, an oxygen atom (-O-), a sulfur atom (-S-), -NH-, $-N(CH_3)-$, $-N(C_2H_5)-$, $-N(C_3H_7)-$, a trivalent nitrogen atom, - C(=O)-, $-S(=O)_2-$, a group obtained by removing 2 to 4 hydrogen atoms from a cycloalkane, a group obtained by removing 2 to 4 hydrogen atoms from an aromatic ring (aryl group), a group

obtained by removing 2 to 4 hydrogen atoms from a heterocycle (heteroaryl group), or a combination thereof. As the aryl group and the heteroaryl group, the groups listed above can be used.

Specific examples of $Z^4$ in general formula (3) that can be used are the same as those listed for $Z^1$. $Z^4$ in general formula (3) is preferably an alkylene group having 2 to 6 carbon atoms, $-C_2H_4-O-C_2H_4-$, $-C_2H4-O-C_2H_4-O-C_2H_4-$, $-C_2H4-O-C_2H_4-O-C_2H4-O-C_2H_4-$, $-C_2H4-O-C_2H_4-O-C_2H4-O-C_2H_4-O-$ $C_2H_4-$, -Ph-, $-Ph-CH_2-Ph-$, -C(=O)-, -C(=O)-O-, -O-C(=O)-, -NH-C(=O)-O-, -O-C(=O)-NH-, -C(=O)-NH-, -NH-C(=O)-, $-S(=O)_2-NH-$, $-NH-S(=O)_2-$, $-S(=O)_2-NH-S(=O)_2-$, or -C(=O)-NH-Ph-, and more preferably an alkylene group having 2 to 6 carbon atoms, $-C_2H4-O-C_2H_4-$, $-C_2H_4-O-C_2H_4-O-C_2H_4-$, $-C_2H_4-O-C_2H_4-O-C_2H_4-O-C_2H_4-$, or $-C_2H_4-O-C_2H_4-O-C_2H_4-O-C_2H_4-O-C_2H_4-$. In addition, -Ph- is a 1,4-phenylene group, a 1,3-phenylene group, a 1,5-phenylene group, or a 1,3,5-substituted phenyl group.

In the artificial viral capsid according to the present invention, the divalent linking group bound to a group derived from a fluorine-containing compound can be bound to the N-terminal side of the β-annulus peptide, but is preferably bound to the C-terminal side of the β-annulus peptide. In an artificial viral capsid of a β-annulus peptide, which is essentially a hollow nanocapsule, the C-terminal side of the β-annulus peptide faces the outside of the nanocapsule. Therefore, by linking a group derived from a fluorine-containing compound to the C-terminal side of the β-annulus peptide, the group derived from the fluorine-containing compound can be presented on the surface of the artificial viral capsid, thereby more efficiently improving cell membrane permeability.

The artificial viral capsid according to the present invention may be linked to another substance at the end of the β-annulus peptide that is not linked to the divalent linking group. The other substance is not particularly limited as long as it does not inhibit the formation of the artificial viral capsid by self-assembly of the β-annulus peptide, and examples thereof include peptides, nucleic acids, low-molecular-weight compounds, chimeric molecules composed of two or more of these, and the like.

The artificial viral capsid according to the present invention has excellent cell membrane permeability due to the presence of groups derived from a fluorine-containing compound on its surface. Therefore, the artificial viral capsid is expected to be used in the pharmaceutical field as a physiologically active substance. For example, the artificial viral capsid according to the present invention is useful as a DDS carrier for transporting a target substance into a cell. For example, the artificial viral capsid according to the present invention is formed by self-assembly in a state in which a target substance to be transported into a cell is linked to the N-terminus of a β-annulus peptide. The artificial viral capsid thus obtained encapsulates a target substance and has its surface modified with a group derived from a fluorine-containing compound, allowing the target substance to be efficiently taken up into cells.

Furthermore, in transfection, the cytotoxicity of the transfection reagent used also affects the efficiency of cellular introduction of the target substance. A reagent with strong cytotoxicity significantly reduces the cell viability after transfection, resulting in low transfection efficiency. In contrast, the artificial viral capsid of the present invention has relatively low cytotoxicity. Therefore, the target substance can be efficiently taken up into various cells without excessively reducing cell viability.

The artificial viral capsid according to the present invention can also encapsulate a target substance to be delivered into a cell as a guest. For example, when the subunits of the artificial viral capsid according to the present invention are self-assembled in an aqueous medium, the target substance can be dissolved or dispersed in the aqueous medium to obtain an artificial viral capsid encapsulating the target substance. The aqueous medium may be water, a buffer such as a phosphate-buffered saline (PBS), or a solution of these containing a small amount of organic solvent.

The target substance to be encapsulated in the artificial viral capsid of the present invention and delivered into a cell is not particularly limited as long as it can be encapsulated in an artificial viral capsid formed from a β-annulus peptide, and examples thereof include substances used for cell labeling, such as fluorescent substances, quantum dots, or the like, and physiologically active substances such as medicinal ingredients or the like. The medicinal ingredient to be encapsulated in the artificial viral capsid of the present invention may be any of peptides, nucleic acids, low-molecular-weight compounds, chimeric molecules composed of two or more of these, or the like. Examples of the nucleic acid include nucleic acids that contribute to RNA interference, such as siRNA, or the like, and nucleic acids for gene recombination and genome editing. In other words, the artificial viral capsid of the present invention can be used as an active ingredient in a DDS carrier composition by encapsulating a target substance therein.

The DDS carrier composition may appropriately contain pharmaceutically acceptable carriers, excipients, additives, and the like in addition to the artificial viral capsid. An example of a suitable carrier is water, which may also be an aqueous solution containing a salt (electrolyte) at a concentration suitable for administration to the human body, or a mixed solvent of water and an organic solvent such as alcohol or the like. Routes of administration include, but are not limited to, oral, intravenous, intraarterial, intramuscular, subcutaneous, transdermal, intraperitoneal, intrathecal, rectal, vaginal, ocular, and inhalation.

By encapsulating a medicinal ingredient, the artificial viral capsid according to the present invention can be used to treat diseases for which the medicinal ingredient provides a therapeutic effect. That is, the artificial viral capsid according to the present invention can be used as an active ingredient in a pharmaceutical composition. A pharmaceutical composition can be prepared by encapsulating a therapeutically effective amount of a medicinal ingredient inside the

artificial viral capsid in the DDS carrier composition. The subjects to which the artificial viral capsid according to the present invention or a composition containing the same is administered are typically individual animals, such as individual mammals, and particularly humans, and administration to ex vivo tissues or cultured cells of these animals may also be performed.

[Examples]

The present invention will be described below with reference to Examples, but the present invention is not limited to these Examples.

The NMR apparatus used for the NMR analysis was JNM-ECZ400S (400 MHz) manufactured by JEOL Ltd., and in $^1$H NMR, tetramethylsilane was used as the reference at 0 ppm, while in $^{19}$F NMR, $C_6F_6$ was used as the reference at -162 ppm.

[Measurement of particle size by dynamic light scattering (DLS)]

The particle size of the artificial viral capsid in each sample was measured by dynamic light scattering. Measurements were performed at 25°C using a dynamic light scattering instrument (DLS, Zetasizer Nano ZS, Malvern) equipped with an incident He-Ne laser (633 nm) and a low-volume glass cuvette cell (ZEN2112, Malvern). During the measurement, the sample diffusion intensity was displayed, and the correlation time of the scattering intensity G($\tau$) was measured 10 times, and the average value was applied to the following equation (1).
[Mathematical formula 1]

$$G(\tau) = B + A \cdot \exp(-2q^2 D\tau) \quad (1)$$

[B: baseline, A: amplitude, q: scattering vector, $\tau$: delay time, D: diffusion coefficient]

The hydrodynamic radius ($R_H$) of the scattering peptide was calculated using the Stokes-Einstein equation shown in the equation (2).
[Mathematical formula 2]

$$R_H = \frac{k_B T}{6\pi\eta D} \quad (2)$$

[$\eta$: solvent viscosity, $k_B$: Boltzmann's constant, T: absolute temperature]

[Observation by transmission electron microscope (TEM)]

The artificial viral capsid in each sample was observed using a TEM (JEM 1400 Plus, manufactured by JEOL). TEM grids (Thin Carbon film TEM grids, manufactured by ALLANCE Biosystems) were hydrophilized using plasma treatment (25°C, 60 Hz, 500 VA, 40 s, JEOL HD Treatment). A sample solution (5 μL) was added to the hydrophilized TEM grid, allowed to stand for 1 minute, and then removed. Next, a stain (5 μL, EM Stainer, Nisshin EM) was added to the TEM grid, allowed to stand for 1 minute, and then removed. After staining, the grid was dried under reduced pressure and observed using a TEM at an accelerating voltage of 80 kV.

[Cell culture]

HepG2 cells were cultured in Dulbecco's modified Eagle's medium (DMEM). All media contained 10% (v/v) fetal bovine serum (FBS), 100 μg/mL streptomycin, 100 units/mL penicillin, 1 mM sodium pyruvate, and 1% (v/v) MEM nonessential amino acids. Cells were maintained at 37°C in a 5% $CO_2$ incubator and subcultured every 3 to 4 days.

[Example 1]

Subunits composed of a β-annulus peptide and a fluoropeptide linked via a bis-maleimide group were self-assembled to form an artificial viral capsid, and the cell membrane permeability was examined.

(1) Synthesis of β-annulus-Cys peptide

Using Fmoc-Ser-Alko-PEG resin (87 mg, 0.24 mmol/g; manufactured by Watanabe Chemical Industries), the

peptide was elongated to the target amino acid sequence by the Fmoc solid-phase synthesis method (H-Ile-Asn(Trt)-His(Trt)-Val-Gly-Gly-Thr(tBu)-Gly-Gly-Ala-Ile-Met-Ala-Pro-Val-Ala-Val-Thr(tBu)-Arg(Pbf)-Gln(Trt)-Leu-Val-Cys(Trt)-Ser(tBu)-Alko-PEG resin; SEQ ID NO: 2). Deprotection (Fmoc removal) was performed by adding 2 mL of a piperidine/DMF solution (piperidine:DMF = 40:60 (volume ratio)) to a column containing the amino acid-incorporated resin. Four equivalents each of Fmoc-functionalized amino acid/DMF solution, HBTU/DMF solution, HOBt. $H_2O$/DMF solution, and DIPEA/NMP solution were added to the resin-containing column, and the condensation reaction was carried out at room temperature for 2 hours. The resulting peptide-attached resin was deprotected (Fmoc removal) by adding piperidine/DMF solution, washed with NMP, and then dried under reduced pressure. Next, a cleavage cocktail (a mixture of TFA/1,2-ethanedithiol/triisopropylsilane/water/thioanisole = 2.58/0.09/0.03/0.15/0.15 (mL)) was added, and the mixture was treated at room temperature for 3 hours to cleave the peptide from the resin and deprotect the protecting groups on the amino acid side chains. The solution from which the peptide was recovered was transferred to a centrifuge tube, and approximately 15 mL of methyl tert-butyl ether (MTBE) was added. The procedure of precipitation by centrifugation (3,500 rpm, 10 minutes) was repeated 3 times, and the recovered precipitate was freeze-dried to obtain the crude peptide as a white solid. The crude peptide was purified by reverse-phase HPLC using a C18 column (Inertsil WP300 C18 column; manufactured by GL Science) with a linear gradient of MeCN/0.1% TFA aqueous solution (5/95 to 100/0 over 100 minutes), and the molecular weight of the target substance was identified by MALDI-TOF-MS (m/z = 2,351, matrix: α-CHCA) to obtain a purified β-annulus-Cys peptide (theoretical yield: 26.3 mg, yield: 18.1 mg, yield rate: 69%). FIG. 1(A) shows the reversed-phase HPLC chart, and FIG. 1(B) shows the MALDI-TOF-MS results.

(2) Synthesis of TMR-β-annulus-Cys peptide

A β-annulus-Cys peptide labeled with a fluorescent dye of tetramethylrhodamine (TMR) (TMR-β-annulus-Cys peptide) was synthesized as follows: First, the target amino acid sequence (H-Ile-Asn(Trt)-His(Trt)-Val-Gly-Gly-Thr(tBu)-Gly-Gly-Ala-Ile-Met-Ala-Pro-Val-Ala-Val-Thr(tBu)-Arg(Pbf)-Gln(Trt)-Leu-Val-Cys(Trt)-Ser(tBu)-Alko-PEG resin) was elongated by the same synthesis as in (1) above, followed by Fmoc removal. After Fmoc removal, 4 equivalents of TMR-COOH, 4 equivalents of COMU, 8 equivalents of DIPEA, and 2-3 mL of NMP were added to the resin and dissolved, followed by stirring for 3 hours in the dark. After the reaction, the solvent was removed and the mixture was washed 5 times with NMP and DCM. Cleavage of the peptide from the resin, deprotection of the amino acid side chain protecting groups, and recovery of the peptide powder were carried out in the same manner as in (1) above to obtain a crude peptide as a red powder. The crude peptide was purified by reverse-phase HPLC in the same manner as in (1) above, and the molecular weight of the target substance was identified by MALDI-TOF-MS (m/z = 2,763, matrix: α-CHCA) to obtain purified a TMR-β-annulus-Cys peptide (theoretical yield: 5 mg, yield: 1 mg, yield rate: 20%). FIG. 2(A) shows the reversed-phase HPLC chart, and FIG. 2(B) shows the results of MALDI-TOF-MS.

(3) Synthesis of β-annulus-$PEG_2$-maleimide

A β-annulus-$PEG_2$-maleimide was synthesized by the following method, in which bismaleimide-$PEG_2$ (1,8-bismaleimido-diethyleneglycol) was bound to the thiol group of the cysteine residue at the second amino acid residue from the C-terminus of the β-annulus-Cys peptide. First, acetonitrile (125 μL) was added to dissolve bismaleimide-$PEG_2$ (1.5 mg). Next, ion-exchanged water (375 μL) was added to dissolve the β-annulus-Cys peptide (1.2 mg). The solution was then added to the bismaleimide-$PEG_2$/MeCN solution and reacted with shaking at 25°C for 24 hours (final concentration: 1 mM β-annulus-Cys peptide, 10 mM bismaleimide-$PEG_2$). The reaction mixture was purified by reversed-phase HPLC in the same manner as in (1) above, and the molecular weight of the target substance was identified by MALDI-TOF-MS (m/z = 2,659, matrix: α-CHCA) to obtain a purified β-annulus-$PEG_2$-maleimide (theoretical yield: 1.33 mg, yield: 0.60 mg, yield rate: 45.1%). FIG. 3(A) shows the reversed-phase HPLC chart, and FIG. 3(B) shows the MALDI-TOF-MS results.

(4) Synthesis of TMR-β-annulus-$PEG_2$-maleimide

A TMR-β-annulus-$PEG_2$-maleimide was synthesized by the following method, in which bismaleimide-$PEG_2$ was bound to the thiol group of the cysteine residue at the second amino acid residue from the C-terminus of the TMR-β-annulus-Cys peptide. First, DMSO (68 μL) was added to dissolve bismaleimide-$PEG_2$ (0.2 mg). Next, ion-exchanged water (612 μL) was added to dissolve the TMR-β-annulus-Cys peptide (0.6 mg). The solution was then added to the bismaleimide-$PEG_2$/DMSO solution and reacted with shaking at 25°C for 12 hours (final concentration: 333 μM TMR-β-annulus-Cys peptide, 1 mM bismaleimide-$PEG_2$). The reaction mixture was purified by reversed-phase HPLC in the same manner as in (1) above, and the molecular weight of the target substance was identified by MALDI-TOF-MS (m/z = 3,071, matrix: α-CHCA) to obtain a purified TMR-β-annulus-$PEG_2$-maleimide (theoretical yield: 166.5 nmol, yield: 14.24 nmol, yield rate: 8.6%). FIG. 4(A) shows the reversed-phase HPLC chart, and FIG. 4(B) shows the MALDI-TOF-MS results.

(5) Synthesis of CAD$^{C8F17}$ F peptide

A CAD$^{C8F17}$ F peptide (H-Cys-Ala-Asp ($C_8F_{17}$)-Phe-$NH_2$) was synthesized as follows.

First, amino acid Asp ($C_8F_{17}$) (D$^{C8F17}$), in which one hydrogen atom of the amino group in the side chain of aspartic acid was substituted with -Ph-$C_8F_{17}$ (a group in which one hydrogen atom of the phenyl group is substituted with a perfluoroalkyl group having 8 carbon atoms), was synthesized.

[Chemical formula 8]

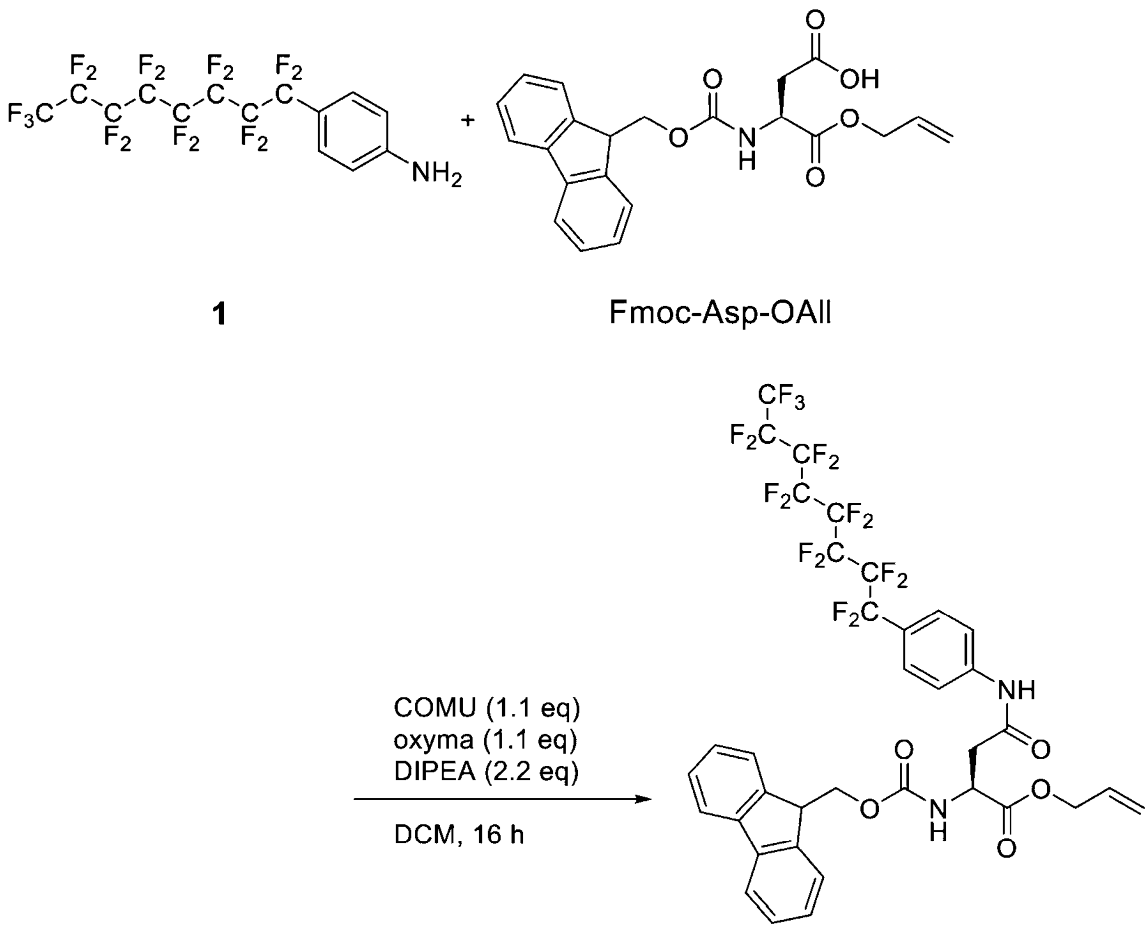


1

Fmoc-Asp-OAll

2a

4-(Perfluorooctyl)aniline (compound (1)) was synthesized according to the method described in Org. Lett., 2019, 21, 6481.

In addition, in a dry 25 mL two-necked flask, 150 mg (0.3 mmol) of compound (1) and 130 mg (0.33 mmol, 1.1 equivalents) of Fmoc-Asp-OAll were dissolved in 6 mL of dichloromethane, and 141 mg (0.33 mmol, 1.1 equivalents) of COMU, 47 mg (0.33 mmol, 1.1 equivalents) of oxyma, and 85.3 mg (0.66 mmol, 2.2 equivalents) of DIPEA were added and stirred at room temperature for 16 hours. The reaction mixture was then quenched with HCl (1N) and extracted 3 times with dichloromethane. The combined organic phase was concentrated under reduced pressure, diluted with ethyl acetate, and washed with HCl (1N), saturated aqueous sodium bicarbonate, and saturated brine. The washed organic phase was dried over sodium sulfate, filtered, and concentrated under reduced pressure to obtain crude product of compound (2a). The crude product was dissolved in acetone and purified by reprecipitation with hexane to obtain pure compound (2a) (Fmoc-Asp ($C_8F_{17}$)-OAll) as a white solid (173 mg, 0.19 mmol) (yield rate: 64.9%).

Compound (2a):

$^1$H NMR (400MHz, DMSO-$D_6$) δ=7.87-7.77 (m,5H), 7.65-7.57 (m,4H), 7.37-7.22 (m,4H), 5.82(m,1H), 5.26 (d, J=17.1Hz,1H), 5.12 (d, J=17.1Hz,1H), 4.54 (m,3H), 4.29-4.18 (m, 3H), 2.94-2.54 (m, 2H).

$^{19}$F NMR (376 MHz, DMSO-$D_6$) δ=-80.0 (s, 3F), -108.8 (s, 2F), -121.0 (s, 2F), -121.6 (s, 6F), -122.3 (s, 2F), -125.7 (s, 2F).

[Chemical formula 9]

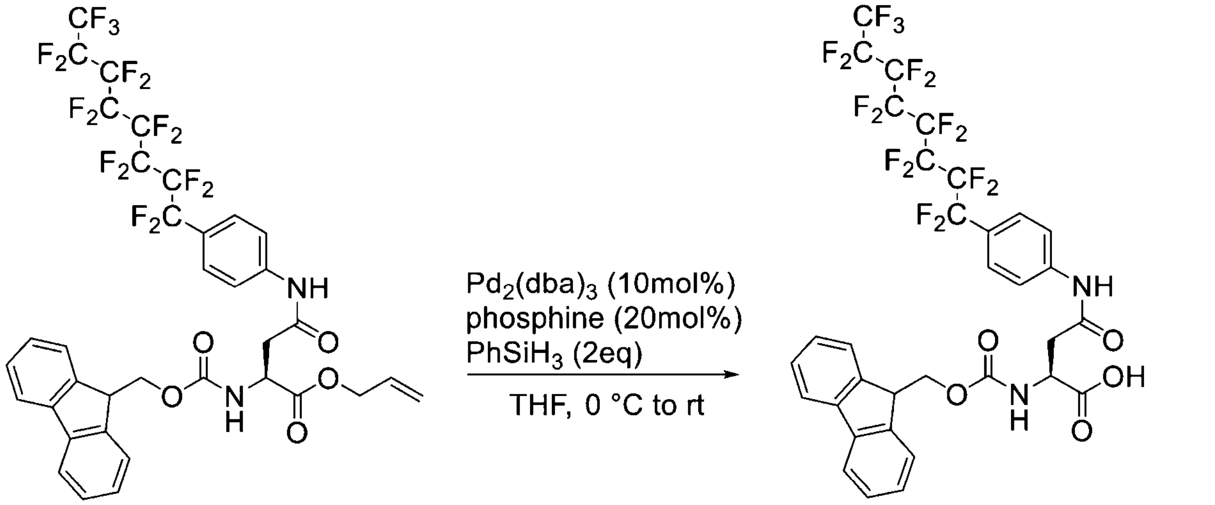


**2a** **2b** **Fmoc-Asp($C_8F_{17}$)-OH**

To a solution of compound (2a) (173 mg, 0.2 mmol) in THF (2 mL), tris(dibenzylideneacetone)dipalladium (0) (10 mol%) and phosphine (20 mol%) were added at 0°C and stirred. Phenylsilane (2 equivalents) was then added, and the mixture was warmed to room temperature and stirred for 2 hours. The reaction mixture was then quenched with 1N HCl (10 mL) and extracted twice with dichloromethane. The organic phase was concentrated under reduced pressure, and the resulting crude product was purified by silica gel column chromatography (chloroform:methanol = 8:1 (volume ratio)) to obtain Fmoc-Asp($C_8F_{17}$)-OH (compound (2b)) as a yellow solid (139 mg, 0.16 mmol, 82% yield).

Compound (2b):

$^{1}$HNMR (500 MHz, DMSO-$D_6$) δ=10.37 (s, 1H), 7.85-7.21 (m, 12H), 4.44 (brs, 1H), 4.52-4.22 (m, 3H), 2.90-2.68 (m, 2H).

$^{19}$F NMR (376 MHz, DMSO-$D_6$) δ=-80.06 (s, 3F), -108.83 (s, 2F), -122.36~-120.96 (m, 10F), -125.41 (s, 2F).

[Chemical formula 10]

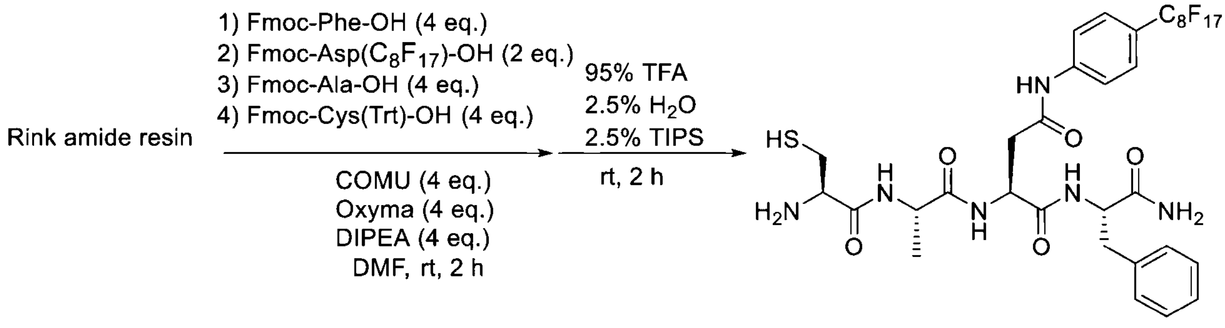


H-Cys-Ala-Asp($C_8F_{17}$)-Phe-$NH_2$

CAD$^{C8F17}$F peptide (16 mg, 17 μmol, yield rate: 22%) was obtained by the Fmoc solid-phase synthesis method using Fmoc-Phe-OH, Fmoc-Asp ($C_8F_{17}$)-OH, Fmoc-Ala-OH, and Fmoc-Cys (Trt)-OH as amino acids to be condensed.

H-Cys-Ala-Asp ($C_8F_{17}$)-Phe-$NH_2$:

LRMS (LC-MS)[M+H]+: m/z calcd for $C_{33}H_{32}F_{17}N_6O_5S^+$ 947.69, found 947.54

(6) Synthesis of β-annulus-CAD$^{C8F17}$ F peptide

[Chemical formula 11]

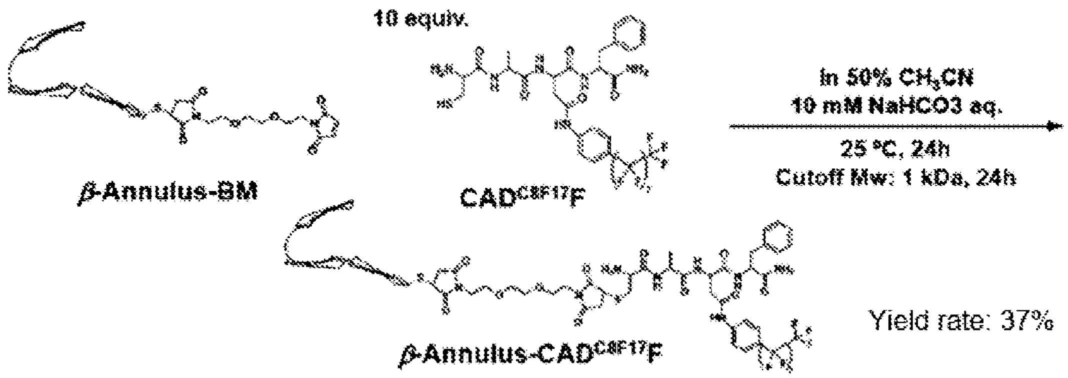


β-Annulus-BM

CAD$^{C8F17}$F

β-Annulus-CAD$^{C8F17}$F

The CAD$^{C8F17}$ F peptide (7.1 mg) was dissolved in 750 μL of MeCN/10 mM $NaHCO_3$ aqueous solution (MeCN/10 mM $NaHCO_3$ = 1/1 (volume ratio)) to prepare an aqueous peptide solution. To this solution, the β-annulus-PEG$_2$-maleimide powder (2.0 mg) was added and the mixture was stirred at 25°C for 24 hours to react (final concentration: 1 mM β-annulus-PEG$_2$-maleimide, 10 mM CAD$^{C8F17}$ F peptide). The reaction mixture was dialyzed against ion-exchanged water for 24 hours (Spectra/por7, cutoff Mw: 1,000, manufactured by Spectrum) and then purified by reversed-phase HPLC using a C4 column (Inertsil WP300 C4 column; manufactured by GL Science) with a linear gradient of MeCN/0.1% TFA aqueous solution (5/95 to 100/0 over 100 minutes). The molecular weight of the target substance was identified by MALDI-TOF-MS (m/z = 3,604, matrix: α-CHCA) to obtain a purified β-annulus-CAD$^{C8F17}$ F peptide (theoretical yield: 2.7 mg, yield: 1.0 mg, yield rate: 37%). FIG. 5(A) shows the reversed-phase HPLC chart, and FIG. 5(B) shows the MALDI-TOF-MS results.

(7) Synthesis of TMR-β-annulus-CAD$^{C8F17}$ F peptide

CAD$^{C8F17}$ F peptide (0.12 mg) was mixed with a TMR-β-annulus-PEG$_2$-maleimide/DMSO solution (100 μL) and reacted with stirring at 25°C for 24 hours (final concentration: 132 μM TMR-β-annulus-PEG$_2$-maleimide, 1,320 μM CAD$^{C8F17}$ F peptide). The reaction mixture was purified by reversed-phase HPLC using a C4 column (Inertsil WP300 C4 column; manufactured by GL Science) with a linear gradient of MeCN/0.1% TFA aqueous solution (5/95 to 100/0 over 100 minutes). The molecular weight of the target substance was identified by MALDI-TOF-MS (m/z = 4017, matrix: α-CHCA) to obtain purified TMR-B-annulus-CAD$^{C8F17}$ F peptide (theoretical yield: 13.2 nmol, yield: 2.1 nmol, yield rate: 15.6%). FIG. 6(A) shows the reversed-phase HPLC chart, and FIG. 6(B) shows the MALDI-TOF-MS results.

(8) Synthesis of TMR-CAD$^{C8F17}$ F peptide

To the CAD$^{C8F17}$ F peptide (0.0946 mg), 1.48 mM TMR-maleimide/DMSO solution (67.6 μL) and 200 mM phosphate buffer (pH7.4, 932.4 μL) were added and stirred at 25°C for 24 hours (final concentration: 100 μMCADC$^{C8F17}$ F peptide, 100 μM TMR-maleimide). The reaction solution was purified by reverse-phase HPLC in the same manner as in (7) above, and the molecular weight of the target substance was identified by MALDI-TOF-MS (m/z = 1,427, matrix: α-CHCA) to obtain a purified TMR-CAD$^{C8F17}$ F peptide (theoretical yield: 100 nmol, yield: 8.49 nmol, yield rate: 8.5%). FIG. 7(A) shows the reversed-phase HPLC chart, and FIG. 7(B) shows the results of MALDI-TOF-MS.

(9) Formation of fluoropeptide-modified capsid

The β-annulus-CAD$^{C8F17}$ F peptide was self-assembled to form a fluoropeptide-modified capsid. Specifically, 1 mM β-annulus-CAD$^{C8F17}$ F peptide (5 μL), DMSO solution (5 μL), and PBS (pH7.4, 90 μL) were mixed and stirred for 30 seconds to form a capsid (final concentration: 50 μM β-annulus-CAD$^{C8F17}$ F peptide in 10% DMSO/PBS (pH7.6)). Furthermore, 1 mM β-annulus-CAD$^{C8F17}$ F peptide (1 μL), DMSO solution (9 μL), and PBS (pH7.4, 90 μL) were mixed and stirred for 30 seconds to form a capsid (final concentration: 10 μM β-annulus-CAD$^{C8F17}$ F peptide in 10% DMSO/PBS (pH7.6) solution).

The resulting capsid was analyzed by DLS to determine the particle size distribution (number-based distribution). The number-average particle diameter of the fluoropeptide-modified capsid formed in a solution containing 50 μM β-annulus-CAD$^{C8F17}$ F peptide was 97 ± 26 nm (FIG. 8(A)). The number-average particle diameter of the fluoropeptide-modified capsid formed in a solution containing 10 μM β-annulus-CAD$^{C8F17}$ F peptide was 81 ± 76 nm (FIG. 8(B)). In addition, TEM images of the fluoropeptide-modified capsid formed in a solution containing 50 μM β-annulus-CAD$^{C8F17}$ F peptide is shown in FIG. 9. In the right panel of FIG. 9, isolated and dispersed fluoropeptide-modified capsids are indicated by arrows.

(10) Formation of TMR-labeled fluoropeptide-modified capsid

The TMR-β-annulus-CAD$^{C8F17}$ F peptide was self-assembled to form a TMR-labeled fluoropeptide-modified capsid. Specifically, 1 mM β-annulus-CAD$^{C8F17}$ F peptide (5 μL), 1 mM TMR-β-annulus-CAD$^{C8F17}$ F peptide (0.5 μL), DMSO solution (5 μL), and PBS (pH7.4, 90 μL) were mixed and stirred for 30 seconds to form a capsid (final concentration: 50 μM β-annulus-CAD$^{C8F17}$ F peptide and 5 μM TMR-β-annulus-CAD$^{C8F17}$ F peptide in 10% DMSO/PBS (pH7.6)).

The resulting capsid was measured by DLS to determine the particle size distribution (number-based distribution). The number-average particle size of the formed TMR-labeled fluoropeptide-modified capsid was 209 ± 61 nm (FIG. 10).

(11) Intracellular introduction of fluoropeptide-modified capsid (part 1)

100 μL of HepG2 cells were added to a single-well glass-bottom dish at a density of 2.0 × $10^4$ cells/well and incubated in a 5% $CO_2$ incubator at 37°C for 24 hours. Next, 100 μL of a DMEM (containing 10% FBS) solution of the TMR-labeled fluoropeptide-modified capsid (50 μM β-annulus-CAD$^{C8F17}$ F peptide and 5 μM TMR-β-annulus-CAD$^{C8F17}$ F peptide), the TMR-labeled fluoropeptide (50 μM β-annulus and 5 μM TMR-CAD$^{C8F17}$ F peptide), or the TMR-labeled capsid (50 μM CAD$^{C8F17}$ F peptide and 5 μM TMR-CAO$^{C8F17}$ F peptide) was added, and the dish was incubated in a 5% $CO_2$ incubator at 37°C for 3 hours. After incubation, the medium was removed and the cells were washed with PBS, followed by addition of 80 μL of the nuclear stain Hoechst 33342 (10 μg/mL) and incubation at 25°C for 10 minutes. After incubation, the nuclear stain solution was removed and the cells were washed with PBS, followed by addition of 100 μL of DMEM (containing 10% FBS) and observation with a confocal scanning microscope (CLSM, FLUOVIEW FV10i, manufactured by Olympus). TMR was detected using the same filter used for rhod-2 detection (Ex: 553 nm, Em: 577 nm, sensitivity: 50%, laser intensity: 15%), while Hoechst 33342 was detected using a blue fluorescence detection filter (Ex: 352 nm, Em: 455 nm, sensitivity: 50%, laser intensity: 15%). The fluorescence intensity of TMR derived from the capsid or the peptide introduced into HepG2 cells was determined using the image analysis software "Image J", by subtracting the fluorescence intensity outside the cells from the fluorescence intensity inside the cells (N=60).

FIG. 11 shows TMR fluorescence images of each cell, merged images of TMR fluorescence images and Hoechst 33342 fluorescence images, and transmitted light images taken with a confocal scanning microscope. In FIG. 11, the upper row shows the cells introduced with the TMR-labeled fluoropeptide-modified capsid, the middle row shows the cells introduced with the TMR-labeled fluoropeptide, and the lower row shows the cells introduced with the TMR-labeled capsid. In addition, FIG. 12 shows the results of measuring the TMR fluorescence intensity of each cell. In FIG. 12, the left column shows the cells introduced with the TMR-labeled fluoropeptide-modified capsid, the center column shows the cells introduced with the TMR-labeled fluoropeptide, and the right column shows the cells introduced with the TMR-labeled capsid.

As shown in FIG. 11, red fluorescence of TMR was observed within the cells introduced with the TMR-labeled fluoropeptide-modified capsid and the cells introduced with the TMR-labeled fluoropeptide, confirming that the capsid was taken up into the cells. In particular, strong TMR fluorescence was observed in the cells introduced with the TMR-labeled fluoropeptide, indicating that the fluoropeptide (CAD$^{C8F17}$ F peptide) itself has the ability to permeate the cell membrane. On the other hand, no TMR fluorescence was observed in the cells introduced with the TMR-labeled capsid, indicating that a capsid whose surface is not modified with a fluoropeptide is hardly taken up into the cells, and that modifying the surface with a fluoropeptide dramatically improves cell membrane permeability. Measurement of the TMR fluorescence intensity of individual cells by image analysis also confirmed that, although the amount of uptake was lower in the cells introduced with the TMR-labeled fluoropeptide-modified capsid than in the cells introduced with the TMR-labeled capsid, there was a significant increase compared to the cells introduced with the TMR-labeled capsid.

(12) Intracellular introduction of fluoropeptide-modified capsid (part 2)

HepG2 cells were seeded at a density of 3.0 × $10^5$ cells/well into a 24-well plate in 500 μL aliquots and cultured for 48 hours in a $CO_2$ incubator. Next, 500 μL of a TMR-labeled fluoropeptide-modified capsid (50 μM β-annulus-CAD$^{C8F17}$ F peptide and 5 μM TMR-β-annulus-CAD$^{C8F17}$ F peptide) solution, a TMR-labeled fluoropeptide (50 μM β-annulus and 5

μM TMR-CAD$^{C8F17}$ F peptide) solution, or a TMR-labeled capsid (50 μM CAD$^{C8F17}$ F peptide and 5 μM TMR-CAD$^{C8P17}$ F peptide) solution was added, and the plates were incubated at 37°C in a 5% $CO_2$ incubator for 3 hours. After incubation, the medium was removed, and the cells were detached from the plates by trypsinization and collected in a 1.5 mL plastic tube to obtain a cell suspension. The resulting cell suspension was centrifuged (800 g, 10 minutes, 4°C) and the supernatant was removed. Then, 500 μL of ice-cold FACS buffer (2% FBS-containing PBS (-)) was added and suspended, followed by centrifugation under the same conditions and removal of the supernatant. This procedure was repeated twice. After the supernatant was removed, 500 μL of FACS buffer was added and the cells were resuspended. Flow cytometry (detection wavelength: 577 nm, Gallios, manufactured by Beckman) was performed 3 times on the resulting cell suspension. As a control, flow cytometry was performed in the same manner on the untreated cells.

2.5 μL of a β-annulus-CAD$^{C8F17}$ F peptide/DMSO stock solution (10 mM), 0.25 μL of a TMR-β-annulus-CAD$^{C8F17}$ F peptide/DMSO stock solution (10 mM), and 122.25 μL of PBS (pH 7.4) were mixed in a 1.5 mL plastic tube, and the resulting solution was dialyzed for 12 hours (Dialysis tube, cutoff Mw: 1000 (1 kDa)) to remove DMSO. Subsequently, 375 μL of DMEM (containing 10% FBS) was added to prepare a TMR-labeled fluoropeptide-modified capsid solution. A TMR-labeled fluoropeptide solution and a TMR-labeled capsid solution were also prepared by dialysis in the same manner.

The dot plots (left) and histograms (right) obtained by flow cytometry are shown in FIG. 13. FIG. 13(A) shows the results of the untreated cells, FIG. 13(B) shows the results of the cells introduced with the TMR-labeled fluoropeptide-modified capsid, FIG. 13(C) shows the results of the cells introduced with the TMR-labeled fluoropeptide, and FIG. 13(D) shows the results of the cells introduced with the TMR-labeled capsid. The histograms were calculated from the areas surrounded by ellipses in the dot plots. In addition, the percentage (%) of the cells that have taken up TMR in each sample is shown in FIG. 14(A), and the median TMR fluorescence intensity for each cell is shown in FIG. 14(B).

As shown in FIG. 13, first, normal cell populations were confirmed in the dot plots of all samples (the areas surrounded by ellipses in the dot plots on the left side of FIG. 13). Upon creating a histogram of the fluorescence intensity of the normal cell population, higher intracellular fluorescence was detected in the cells introduced with the TMR-labeled fluoropeptide-modified capsid and the cells introduced with the TMR-labeled fluoropeptide compared to the untreated cells and the cells introduced with the TMR-labeled capsid (FIG. 13, right). As shown in FIG. 14, it was confirmed that the TMR-labeled fluoropeptide was taken up in approximately 90% of the cells introduced with the TMR-labeled fluoropeptide-modified capsids, and although the intracellular fluorescence intensity was lower than that of the cells introduced with the TMR-labeled fluoropeptides, it was significantly higher than that of the cells introduced with the TMR-labeled capsids. These results also demonstrate that modifying the capsid surface with a fluoropeptide improves the cell membrane permeability and increases intracellular introduction efficiency.

[Example 2]

Drugs were encapsulated in the fluoropeptide-modified capsid, and the efficiency of intracellular delivery was examined. The β-annulus-CAD$^{C8F17}$ F peptide synthesized in Example 1 was used as the subunit for the fluoropeptide-modified capsid. Doxorubicin (Dox) was used as the drug. For comparison, the β-annulus-Cys peptide synthesized in Example 1 was used as the subunit for capsid formation that was not modified with a fluoropeptide.

(1) Formation of Dox-encapsulated fluoropeptide-modified capsid

A 10 mM Dox/DMSO solution (1 μL) was mixed with a 10 mM β-annulus-CAD$^{C8F17}$ F peptide/DMSO solution (1 μL), followed by the addition of PBS (pH 7.4, 98 μL) and stirring for 30 seconds (final concentration: 100 μM Dox, 100 μM β-annulus-CAD$^{C8F17}$ F peptide). The resulting solution was dialyzed for 12 hours (Dialysis tube, cutoff Mw: 1000 (1 kDa)) to remove free Dox and DMSO, yielding a Dox-encapsulated fluoropeptide-modified capsid. The Dox concentration after encapsulation (Dox binding rate (%)) was calculated by measuring the UV-vis spectra of the dialyzed solution using a Nanodrop ultraspectrophotometer (manufactured by Thermo Scientific) (molar extinction coefficient of Dox $\varepsilon_{480}$ =11,500 $M^{-1}cm^{-1}$) (Non-Patent Document 5). A similar procedure was performed using the β-annulus-Cys peptide to obtain a Dox-encapsulated fluoropeptide-unmodified capsid that was not modified with a fluoropeptide.

The resulting Dox-encapsulated fluoropeptide-modified capsid was analyzed by DLS to determine the particle size distribution (number-based distribution), revealing a number-average particle diameter of 78 ± 26 nm (FIG. 15). In addition, a TEM image of the Dox-encapsulated fluoropeptide-modified capsid is shown in FIG. 16. As shown in FIGS. 15 and 16, the β-annulus-CAD$^{C8F17}$ F peptide stably self-assembled to form a capsid, even when Dox was encapsulated.

Furthermore, the UV-Vis spectra and Dox binding rates (%) of the Dox-encapsulated fluoropeptide-modified capsid and the Dox-encapsulated fluoropeptide-unmodified capsid are shown in FIG. 17. The UV-Vis measurement results before and after dialysis were compared to calculate the Dox binding rate for each sample. The Dox binding rate for the Dox-encapsulated fluoropeptide-modified capsid was 41%, while the Dox binding rate for the Dox-encapsulated fluoropeptide-unmodified capsid was 14%. These results confirmed that the fluoropeptide-modified capsid is useful as a

DDS carrier for encapsulating drugs such as Dox.

(2) Cytotoxicity evaluation of Dox-encapsulated fluoropeptide-modified capsid

The cytotoxicity of the Dox-encapsulated fluoropeptide-modified capsid and the Dox-encapsulated fluoropeptide-unmodified capsid prepared in (1) above was examined by WST-assay to determine whether Dox taken up into the cells by the fluoropeptide-modified capsid exerts its pharmacological effect.

First, the dialyzed Dox-encapsulated fluoropeptide-modified capsid solution and the Dox-encapsulated fluoropeptide-unmodified capsid solution prepared in (1) above were each diluted 4-fold with DMEM (+) to prepare diluted capsid solutions.

HepG2 cells were seeded in 100 μL aliquots into a 96-well plate at a density of $2.0 \times 10^4$ cells/well and cultured at 37°C for 24 hours in a $CO_2$ incubator. The medium was then removed, and 100 μL of the diluted capsid solution was added per well and cultured at 37°C for 24 hours in a $CO_2$ incubator. After culture, the culture supernatant was removed, and a viable cell count measurement solution (a mixed solution prepared by diluting 10 μL of Cell Counting Kit with 8 μL of medium) was added and cultured for 4 hours. The supernatant was then collected and diluted 10-fold, followed by UV-vis spectroscopy using a Nanodrop, and cell viability was calculated from the absorbance at a wavelength of 460 nm (N=3). For comparison, cell viability was calculated in the same manner for the cells cultured in a solution of Dox alone dissolved in DMEM(+), or a solution of Dox and the $CAD^{C8F17}$ F peptide synthesized in Example 1 dissolved in DMEM(+). The Dox concentration in each sample was set to 0.1 μM for comparison. The measurement results of cell viability (%) are shown in FIG. 18.

Cytotoxicity evaluation revealed that the cells introduced with Dox alone as a control ("Dox" in the figure), the cells introduced with Dox and the $CAD^{C8F17}$ F peptide ("2.4 μM $CAD^{C8F17}$ F + Dox" in the figure), and the cells introduced with the Dox-encapsulated fluoropeptide-unmodified capsid ("7.1 μM β-Annulus + Dox" in the figure) all showed a cell viability of 85% or higher, and virtually no toxicity was observed. In contrast, the cells introduced with the Dox-encapsulated fluoropeptide-modified capsid ("2.4 μM β-annulus-$CAD^{C8F17}$ F + Dox" in the figure) showed a low cell viability of 65%, demonstrating cytotoxicity (FIG. 18). These results confirmed that even at a low Dox concentration of 0.1 μM, encapsulation of Dox into the fluoropeptide-modified capsid resulted in cytotoxicity due to Dox. These results demonstrate that the fluoropeptide-modified capsid is useful as an anticancer drug transport carrier.

[Example 3]

To investigate the intracellular uptake pathway of the fluoropeptide-modified capsid, cellular uptake experiments were performed in the presence of an endocytosis inhibitor. As the fluoropeptide-modified capsid, the TMR-labeled fluoropeptide-modified capsid prepared in Example 1 (50 μM β-annulus-$CAD^{C8F17}$ F peptide and 5 μM TMR-β-annulus-$CAD^{C8F17}$ F peptide) was used.

First, 100 μL of HepG2 cells were added to a single-well glass-bottom dish at a density of $2.0 \times 10^4$ cells/well and incubated in a 5% $CO_2$ incubator at 37°C for 24 hours. Next, 60 μL of DMEM (containing 10% FBS) containing 80 μM EIPA (5-(N-ethyl-N-isopropyl)-amiloride, a macropinocytosis inhibitor), 20 μM Pitstop 2 (N-[5-(4-bromobenzylidene)-4-oxo-4,5-dihydro-1,3-thiazol-2-yl]naphthalene-1-sulfonamide, a clathrin-mediated endocytosis inhibitor), and 160 μM Genistein (5,7-dihydroxy-3-(4-hydroxyphenyl)-4H-1-benzopyran-4-one, a caveolae-mediated endocytosis inhibitor) was added to each well, and the cells were incubated at 37°C in a 5% $CO_2$ incubator for 30 minutes. The medium was then removed, and the cells were washed with PBS(-). Next, 60 μL of DMSO-containing DMEM (containing 10% FBS and 5% DMSO) containing either 80 μM EIPA, 20 μM Pitstop 2, or 160 μM Genistein and the TMR-labeled fluoropeptide-modified capsid (50 μM β-annulus-$CAD^{C8F17}$ F peptide and 5 μM TMR-β-annulus-$CAO^{C8F17}$ F peptide) was added to each well, and the cells were incubated for 3 hours at 37°C in a 5% $CO_2$ incubator. As a control, the cells were similarly incubated for 3 hours in a 5% $CO_2$ incubator at 37°C with a medium of DMSO-containing DMEM (containing 10% FBS and 5% DMSO) that only contained the TMR-labeled fluorinated peptide-modified capsid. After incubation, the medium was removed and the cells were washed with PBS, after which 80 μL of the nuclear stain Hoechst 33342 (10 μg/mL) was added and incubated for 10 minutes at 25°C. After incubation, the cells were washed with PBS and stained with the nuclear stain Hoechst 33342, followed by observation with a confocal scanning microscope to determine the fluorescence intensity of TMR derived from the capsid introduced into the HepG2 cells, in the same manner as in Example 1.

FIG. 19 shows TMR fluorescence images of each cell captured with a confocal scanning microscope. FIG. 20 also shows the results of measuring the intracellular TMR fluorescence intensity of each cell. The P value of the TMR fluorescence intensity was calculated using the Kruskal-Wallis test (Dunn test). As shown in FIGS. 19 and 20, it was confirmed that intracellular red fluorescence was significantly reduced in the cells cultured in the medium containing Genistein as an inhibitor of caveolae-dependent endocytosis and the TMR-labeled fluorinated peptide-modified capsid. These results suggest that the fluorinated peptide-modified capsid is primarily taken up into the cells via caveolae-dependent endocytosis.

[Example 4]

A β-annulus-CAD$^{C6F13}$ F peptide was self-assembled to form a CAD$^{C6F13}$ F peptide-modified artificial viral capsid, and the cell membrane permeability was examined.

(1) Synthesis of β-annulus-CAD$^{C6F13}$ F peptide

[Chemical formula 12]

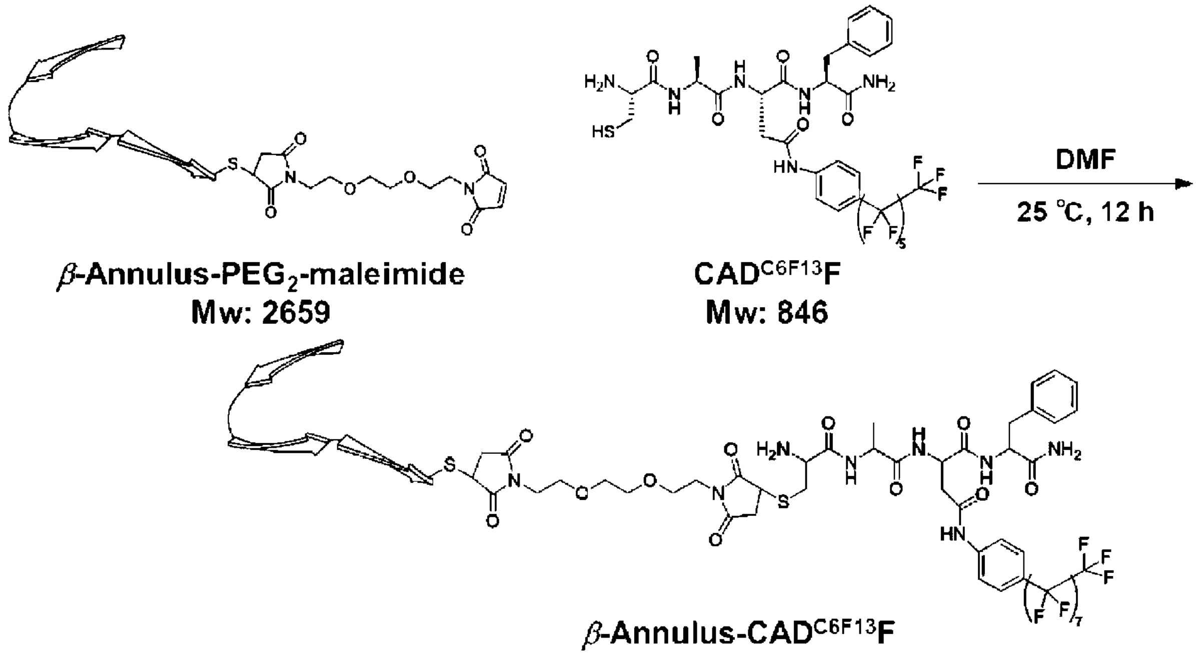


*β*-Annulus-PEG$_2$-maleimide
Mw: 2659

CAD$^{C6F13}$F
Mw: 846

*β*-Annulus-CAD$^{C6F13}$F
Mv: 3505

A 10 mM DMF solution of the β-annulus-PEG$_2$-maleimide (2.5 μL), a 10 mM DMF solution of the CAD$^{C6F13}$ F peptide (2.5 μL), and 35 μL of DMF were mixed and reacted at 25°C for 24 hours with stirring. The DMF in the reaction solution was removed using a concentrator (Convenience Evaporator; manufactured by BioChromato), and the residue was then dissolved in 50 μL of trifluoroethanol. The resulting solution was purified by reverse-phase HPLC using a C4 column (Inertsil WP300 C4 column; manufactured by GL Science) with a linear gradient of MeCN/0.1% TFA aqueous solution (5/95 to 100/0 in 100 minutes), and the molecular weight of the target substance was identified by ESI-MS (m/z = 877.1357, Calcd. $[M+4H]^{4+}$ = 877.1362) to obtain the purified β-annulus-CAD$^{C6F13}$ F peptide (theoretical yield: 1.75 mg, yield: 0.2 mg, yield rate: 27%). FIG. 21(A) shows the reversed phase HPLC chart, and FIG. 21 (B) shows the results of ESI-MS.

(2) Preparation of mRNA-encapsulated artificial viral capsid and intracellular introduction

[Chemical formula 13]

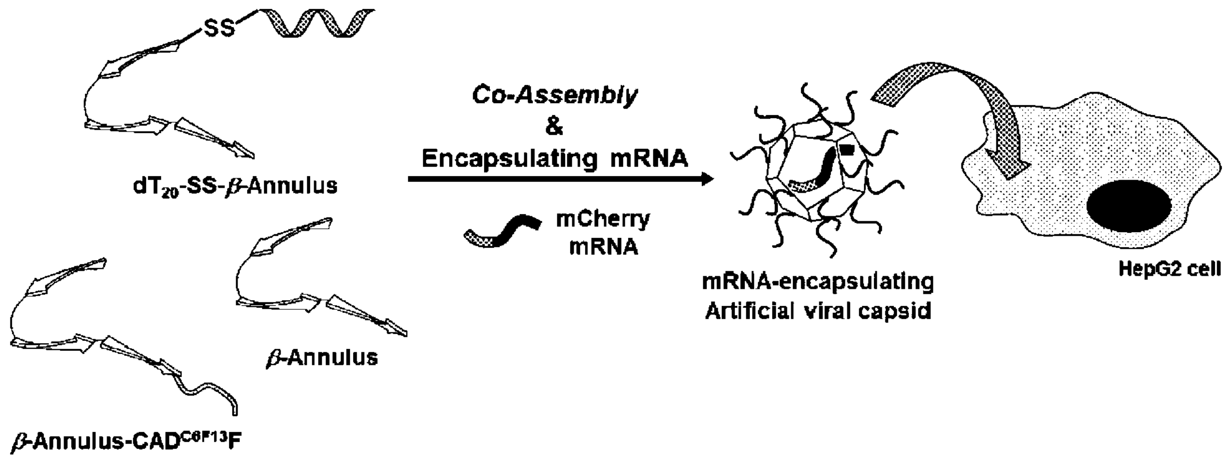


A $dT_{20}$-SS-β-annulus peptide conjugate was prepared as described in Applied Sciences, 2020, 10, 8004. An Eppendorf tube was charged with 1 μL of a 500 μM β-annulus-$CAD^{C6F13}$ F peptide solution, 1.28 μL of a 500 μM unmodified β-annulus peptide solution, and 11 μL of a 10 μM dT20-SS-β-annulus solution, followed by lyophilization. 20 μL of 1.1 μM mCherry mRNA (manufactured by OZBIOSCIENCES) dissolved in nuclease-free water was added to the resulting peptide powder, followed by slow pipetting. The mixture was then incubated in a constant temperature bath at 37°C for 30 minutes to produce artificial viral capsids encapsulating $CAD^{C6F13}$ F peptide-modified mRNA.

50 μL of each prepared sample was seeded onto a HepG2 cell-immobilized dish (cell density: $4.0 \times 10^4$ cells/100 μL) and incubated at 37°C in 5% $CO_2$ for 3 hours. After removing the solvent and washing with PBS, 80 μL of 1 μg/mL "Hoechst 33342 in DMEM" medium (containing 10% FBS) was added and incubated at 37°C in 5% $CO_2$ for 10 minutes. After removing the solvent and washing with PBS, 100 μL of DMEM medium (containing 10% FBS) was added, and the cells were observed under a confocal scanning microscope (CLSM, FLUOVIEW FV10i, manufactured by Olympus). To detect mCherry expressed in HepG2 cells, Ex: 584 nm, Em: 610 nm, sensitivity: 50%, laser intensity: 15% were used, and to detect Hoechst 33342, Ex: 352 nm, Em: 455 nm, sensitivity: 50%, laser intensity: 15% were used. The fluorescence intensity of mCherry expressed in HepG2 cells was determined by subtracting the fluorescence intensity outside the cells from the fluorescence intensity inside the cells using the image analysis software "Image J" (N=60).

FIG. 22 shows the Hoechst 33342 fluorescent images, mCherry fluorescent images, merged images of mCherry images and Hoechst 33342 fluorescent images, and transmitted light images of each cell, all captured with a confocal scanning microscope. FIG. 23 also shows the results of measuring the mCherry fluorescent intensity of each cell. In FIG. 23, the left column shows the cells introduced with mCherry mRNA using a transfection reagent (Lipofectamine MessengerMAX), the center column shows the cells introduced with mCherry mRNA using the $CAD^{C6F13}$ F peptide-modified artificial viral capsid, and the right column shows the cells introduced with mCherry mRNA using the unmodified artificial viral capsid.

As shown in FIG. 22, in the cells introduced with mCherry mRNA using the transfection reagent and $CAD^{C6F13}$ F peptide-modified artificial viral capsid, red fluorescence of mCherry was observed within the cells, confirming that the mRNA was taken up into the cells and that mCherry was expressed.

Furthermore, as shown in FIG. 23, when mCherry mRNA was introduced using the $CAD^{C6F13}$ F peptide-modified artificial viral capsid, the fluorescence intensity was slightly lower than when the transfection reagent was used, but was approximately the same. Furthermore, the fluorescence intensity was approximately 5 times higher than that observed when mCherry mRNA was introduced using the unmodified artificial viral capsid. These results confirm that modifying an artificial viral capsid with $CAD^{C6F13}$ F enables sufficient delivery and expression of mRNA into cells.

## Claims

1. An artificial viral capsid formed by self-assembly of multiple subunits, wherein the subunit comprises:

   a β-annulus peptide of tomato bushy stunt virus,
   a group derived from a fluorine-containing compound, and
   a divalent linking group that links the β-annulus peptide to the group derived from a fluorine-containing compound;

and
the divalent linking group is linked to a C-terminus of the β-annulus peptide.

2. The artificial viral capsid according to claim 1, wherein the fluorine-containing compound is a fluoropeptide.

3. The artificial viral capsid according to claim 2, wherein at least one side chain of amino acid residues constituting the fluoropeptide is represented by the following general formula (1):

[Chemical formula 1]

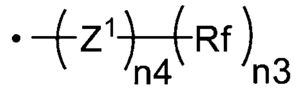

(1)

[wherein $Z^1$ is a linking group other than a di-, tri-, or tetra-valent alkylene group; Rf is a $C_{1-30}$ alkyl group substituted with at least two fluorine atoms (when the $C_{1-30}$ alkyl group has two or more carbon atoms, it may have 1 to 5 ether-bonding oxygen atoms between the carbon atoms), $-SF_5$, or-$SF_4$-$CR^{101}R^{102}$-$CR^{103}R^{104}Cl$ ($R^{101}$, $R^{102}$, $R^{103}$, and $R^{104}$ are each independently a hydrogen atom, a fluorine atom, or a chlorine atom, and two or more of $R^{101}$, $R^{102}$, $R^{103}$, and $R^{104}$ are fluorine atoms); n3 is 1, 2, or 3; n4 is 0 or 1; and a black dot represents a binding site.]

4. The artificial viral capsid according to claim 3, wherein Rf is represented by the following general formula (f-1) or (f-2):

[Chemical formula 2]

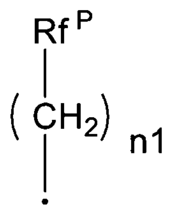

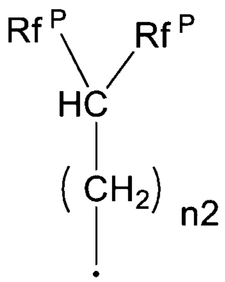

(f-1) (f-2)

[In the formula, $Rf^P$ represents a fully halogenated $C_{1-10}$ alkyl group containing at least two fluorine atoms (when the fully halogenated $C_{1-10}$ alkyl group has two or more carbon atoms, it may have an ether-bonding oxygen atom between the carbon atoms), n1 represents an integer of 0 to 10, n2 represents an integer of 0 to 9, and a black dot represents a binding site.]

5. The artificial viral capsid according to claim 3, wherein the amino acid residue having a group represented by general formula (1) as a side chain is an amino acid residue having 1 to 3 Rfs linked directly or indirectly to a side chain of a natural amino acid.

6. The artificial viral capsid according to claim 1, wherein the divalent linking group is a bis-maleimide group containing maleimide groups at both ends.

7. The artificial viral capsid according to claim 1, wherein

the β-annulus peptide has a cysteine residue at or near the C-terminus, and
the divalent linking group is linked to a thiol group derived from the cysteine residue.

8. A pharmaceutical composition comprising the artificial viral capsid according to any one of claims 1 to 7.

**9.** A drug delivery carrier composition comprising the artificial viral capsid according to any one of claims 1 to 7.

FIG. 1

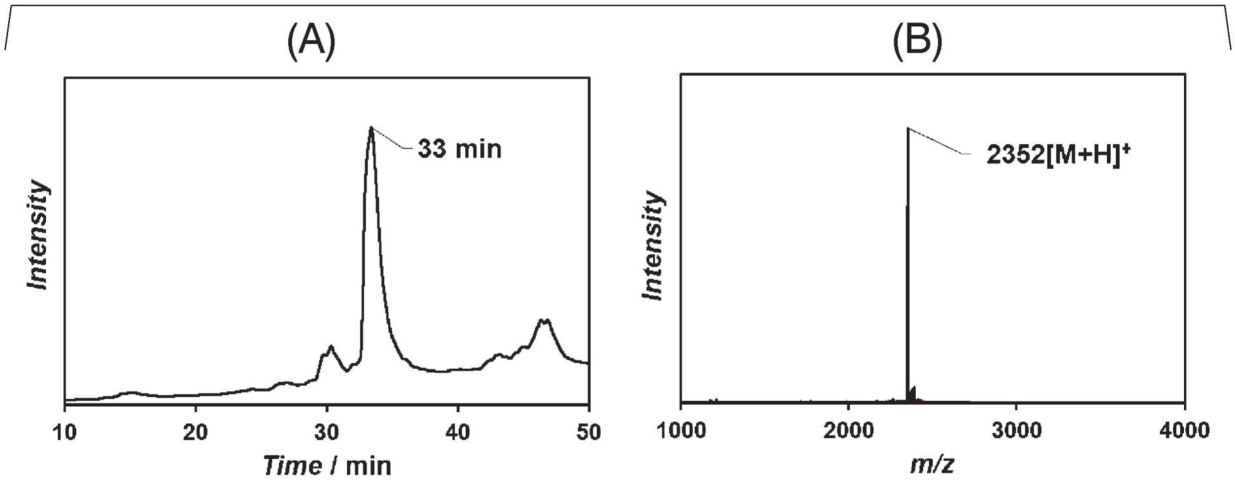


FIG. 2

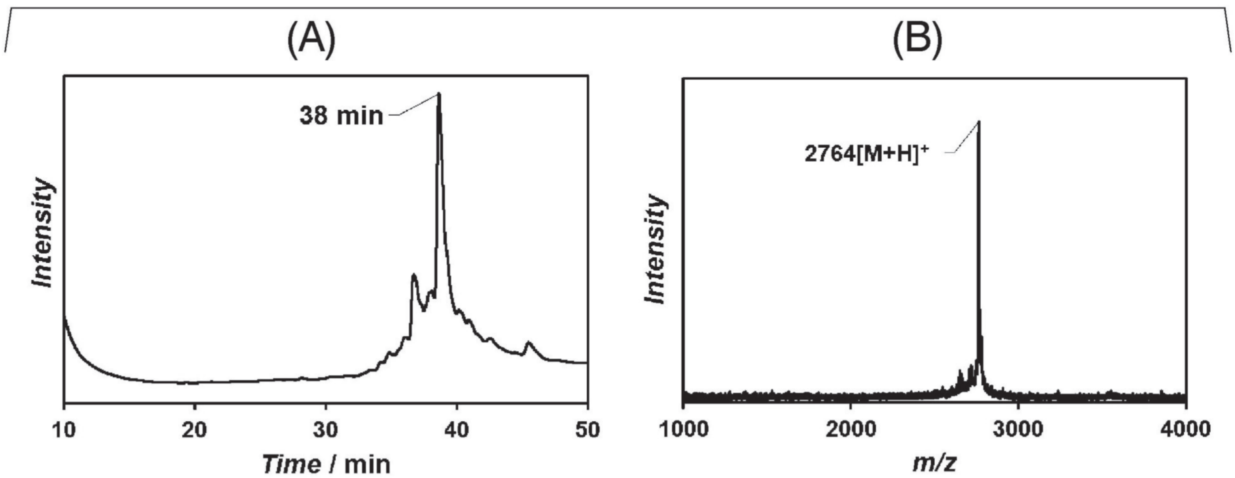


FIG. 3

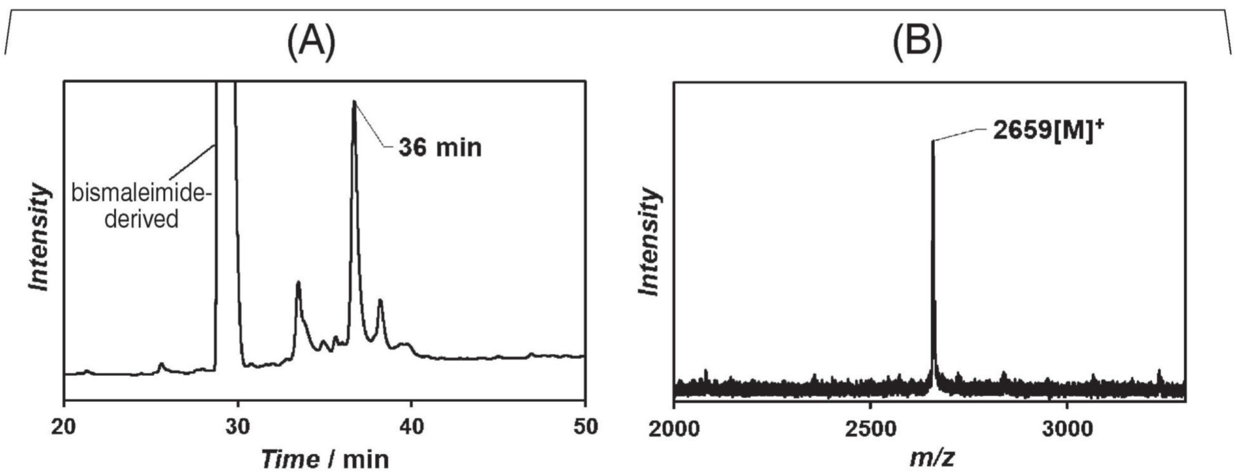

FIG. 4

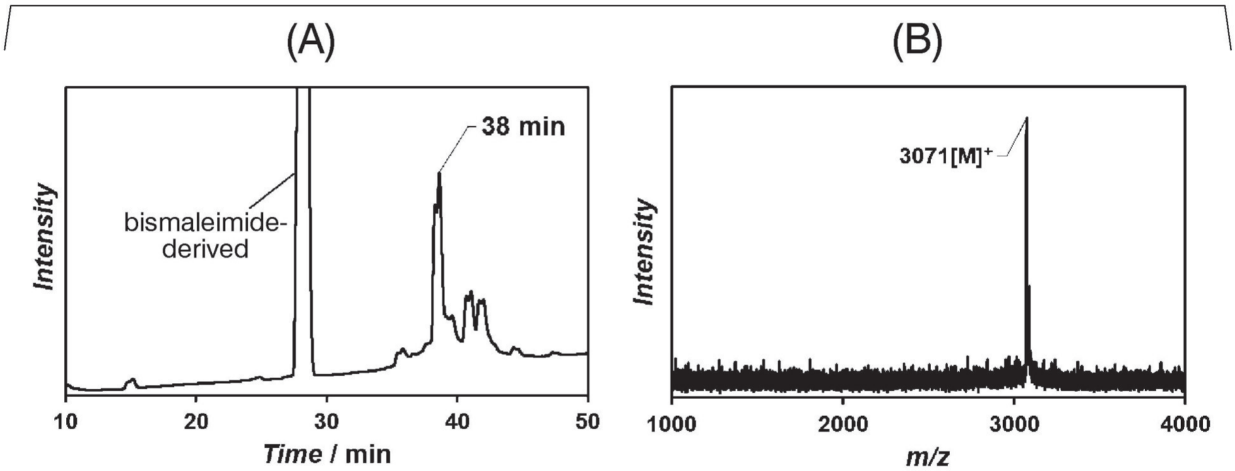


FIG. 5

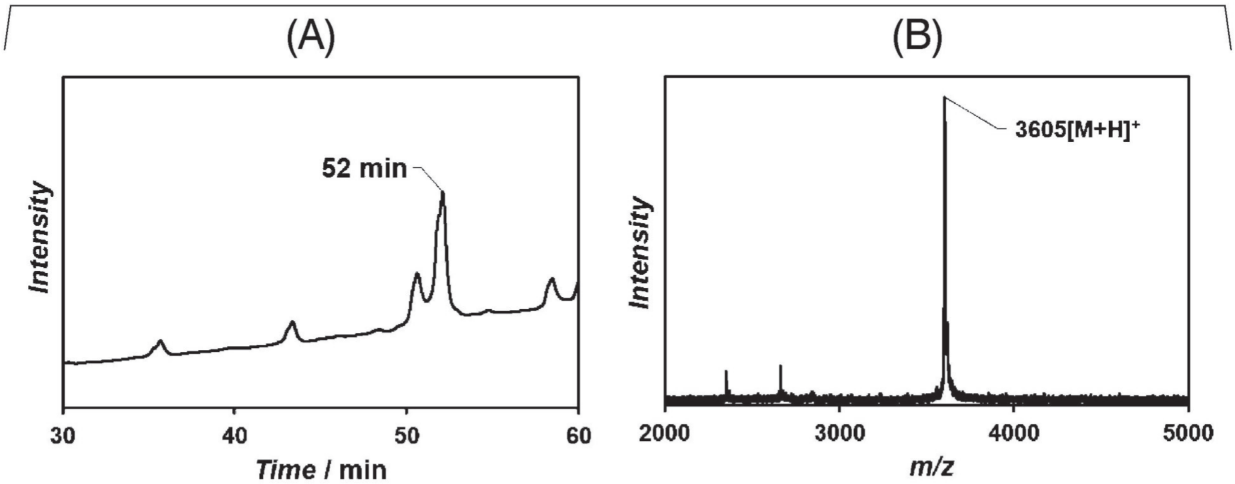


FIG. 6

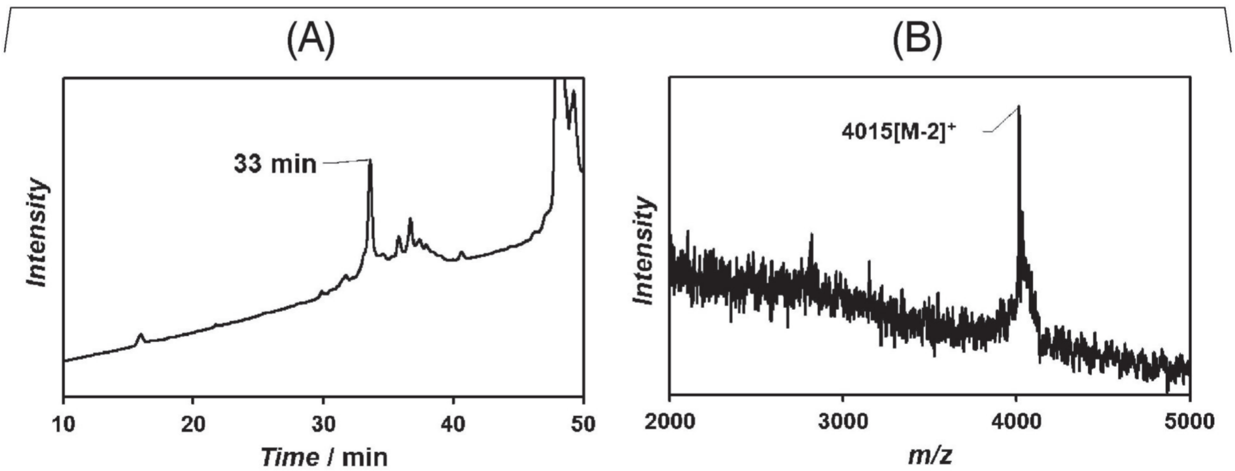

FIG. 7

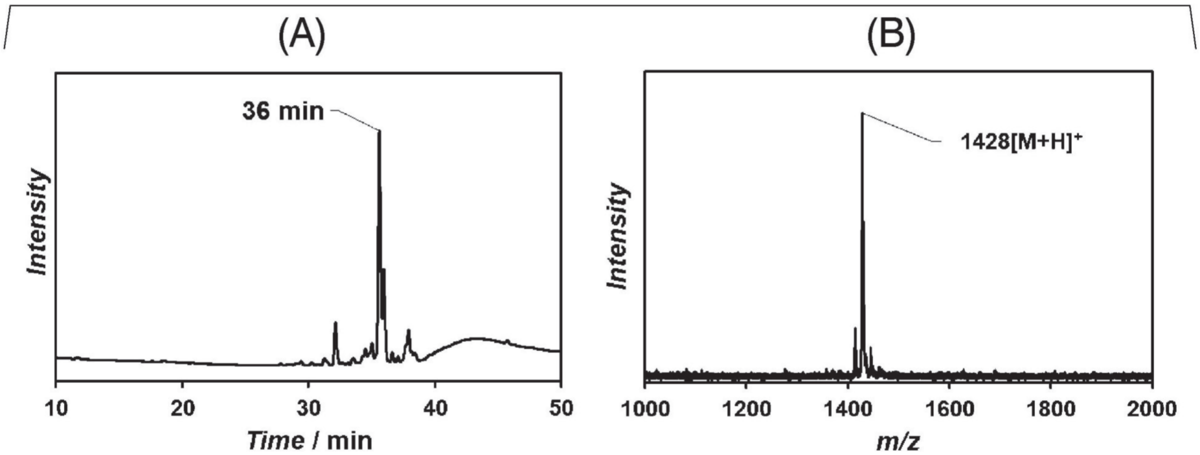


FIG. 8

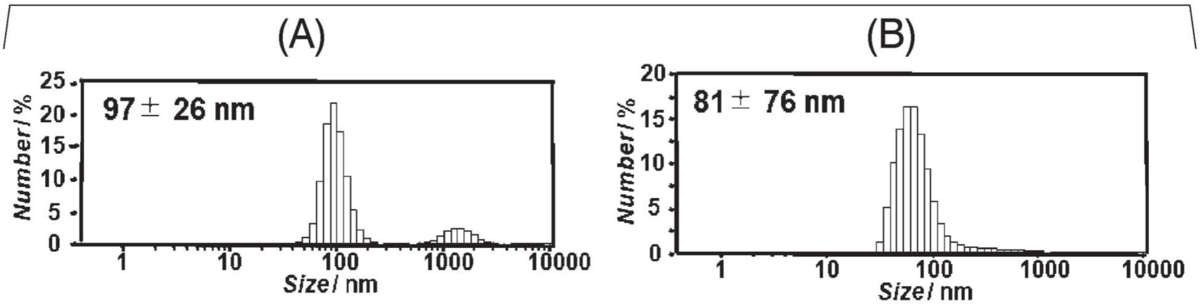


FIG. 9

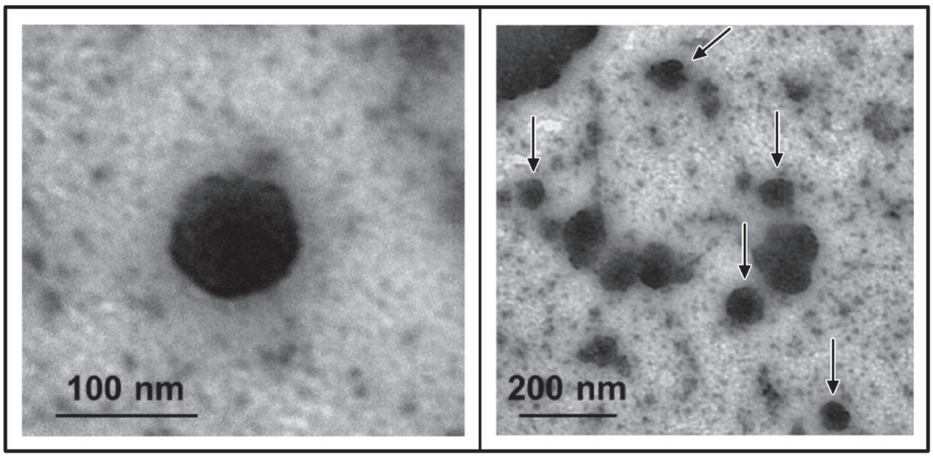


FIG. 10

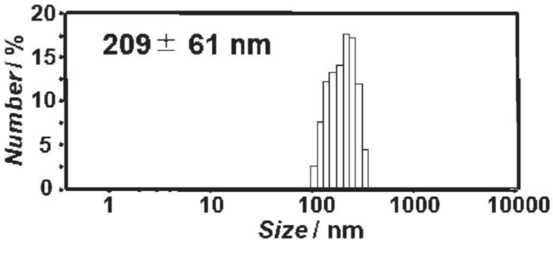

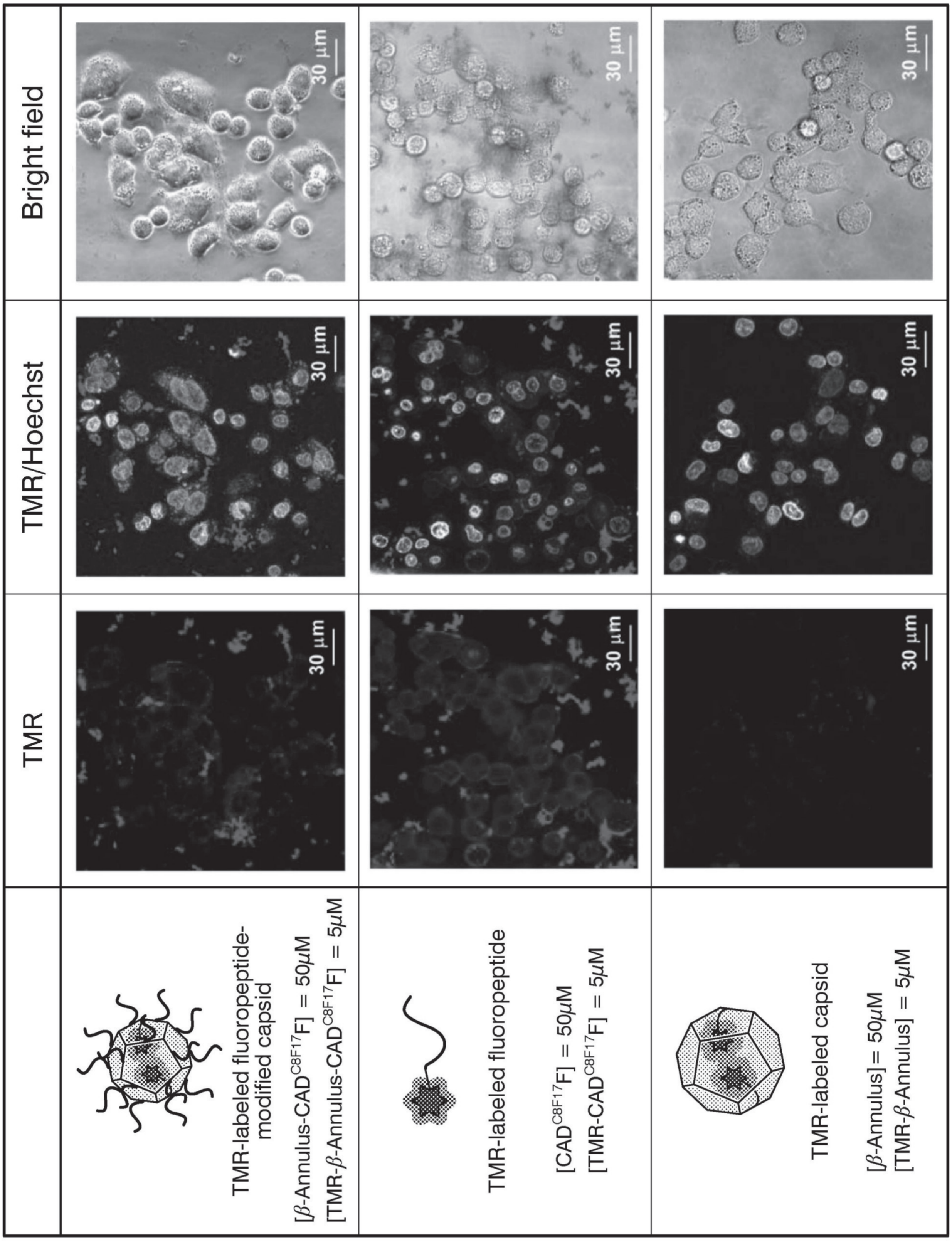
TMR
TMR/Hoechst
Bright field
30 μm
TMR-labeled fluoropeptide-modified capsid
[β-Annulus-CAD$^{C8F17}$F] = 50μM
[TMR-β-Annulus-CAD$^{C8F17}$F] = 5μM
TMR-labeled fluoropeptide
[CAD$^{C8F17}$F] = 50μM
[TMR-CAD$^{C8F17}$F] = 5μM
TMR-labeled capsid
[β-Annulus]= 50μM
[TMR-β-Annulus] = 5μM

FIG. 11

FIG. 12

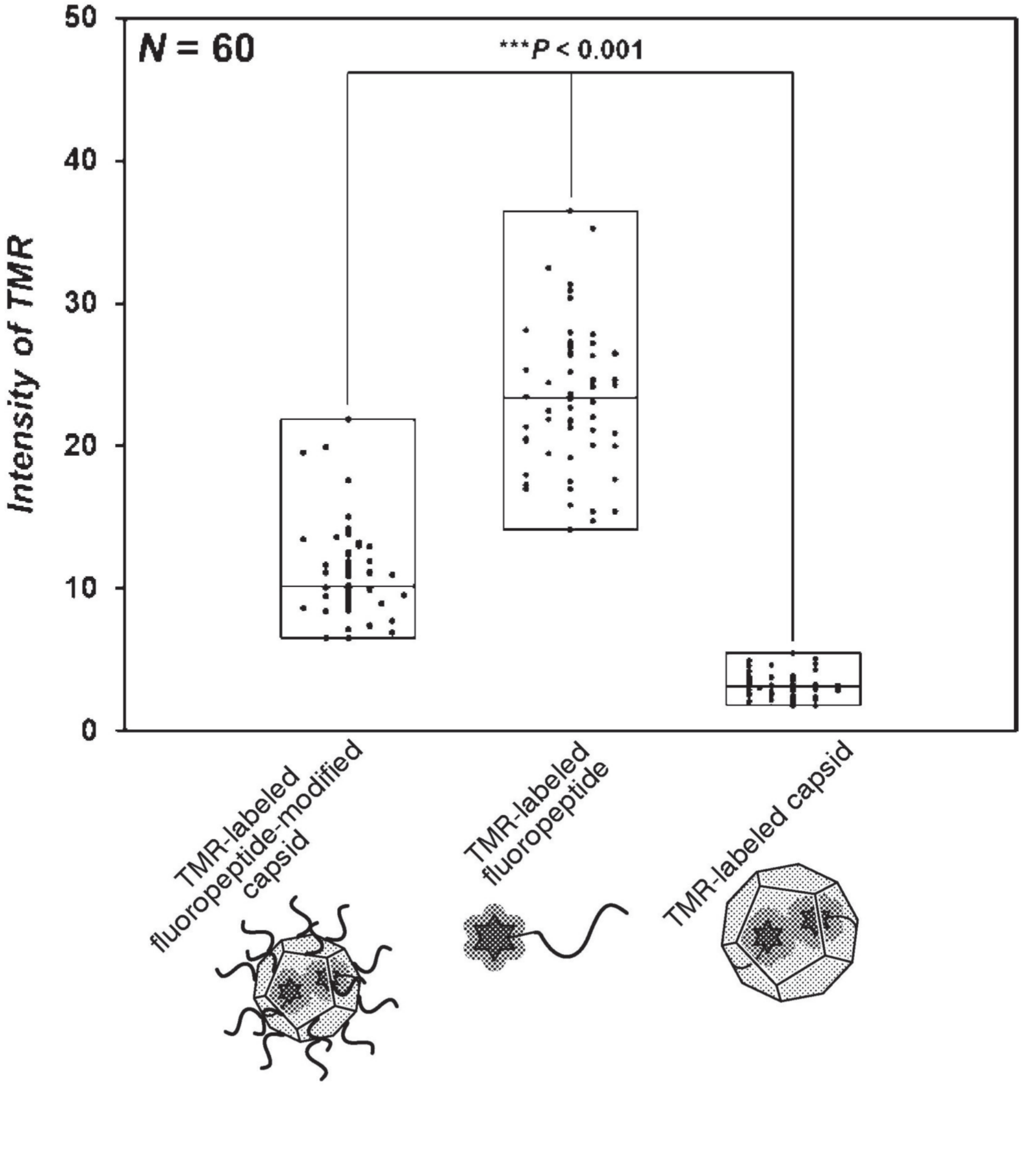
N = 60
***P < 0.001
Intensity of TMR
50
40
30
20
10
0
TMR-labeled fluoropeptide-modified capsid
TMR-labeled fluoropeptide
TMR-labeled capsid

FIG. 13

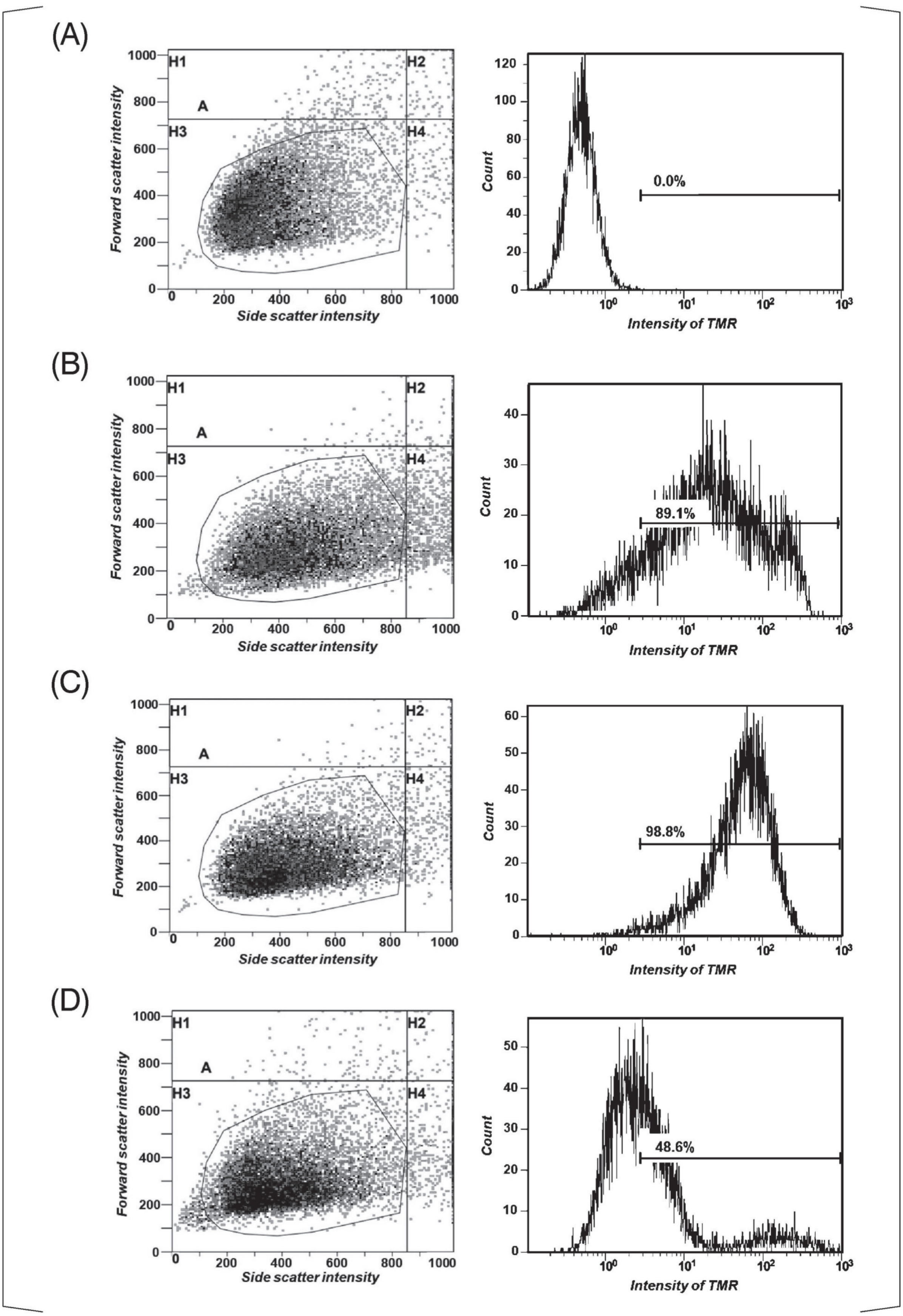
(A)
H1
H2
A
H3
H4
Forward scatter intensity
Side scatter intensity
Count
0.0%
Intensity of TMR
(B)
89.1%
(C)
98.8%
(D)
48.6%

FIG. 14

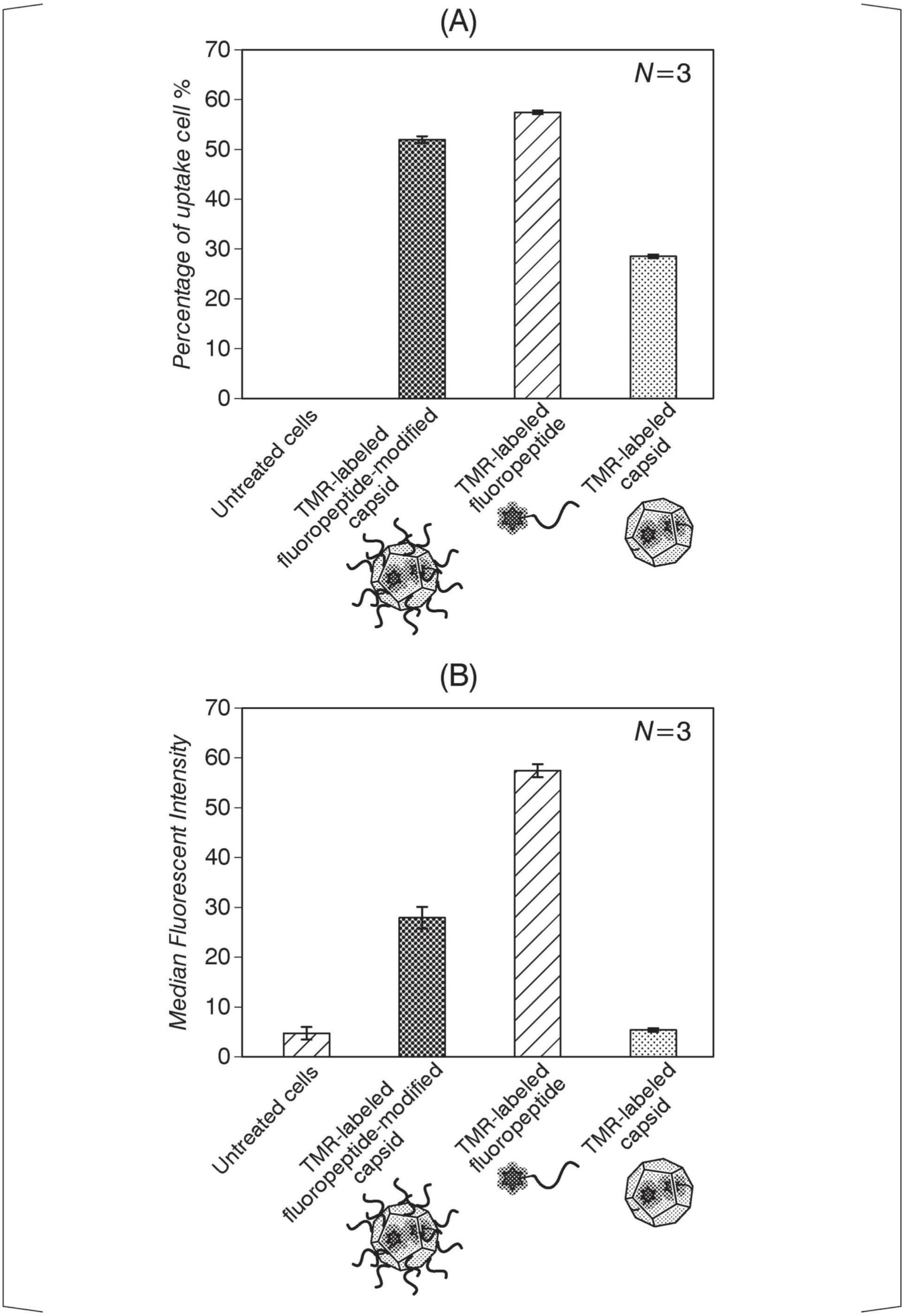
(A)
N=3
Percentage of uptake cell %
0
10
20
30
40
50
60
70
Untreated cells
TMR-labeled fluoropeptide-modified capsid
TMR-labeled fluoropeptide
TMR-labeled capsid
(B)
N=3
Median Fluorescent Intensity
0
10
20
30
40
50
60
70
Untreated cells
TMR-labeled fluoropeptide-modified capsid
TMR-labeled fluoropeptide
TMR-labeled capsid

*FIG. 15*

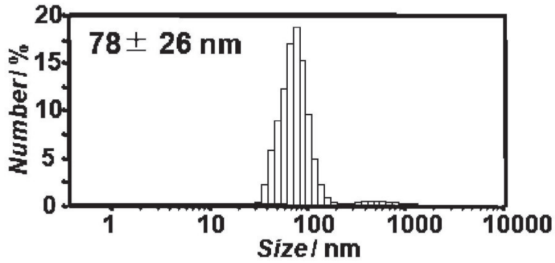


*FIG. 16*

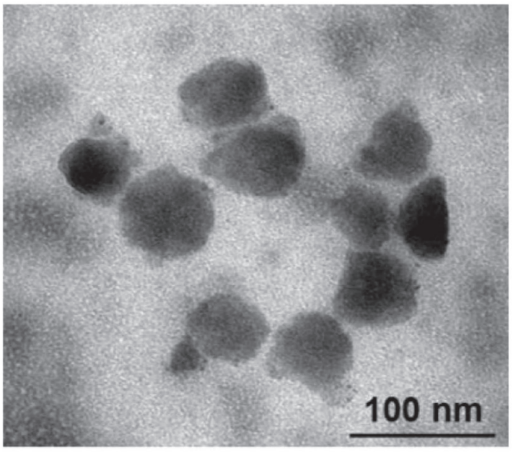


*FIG. 17*

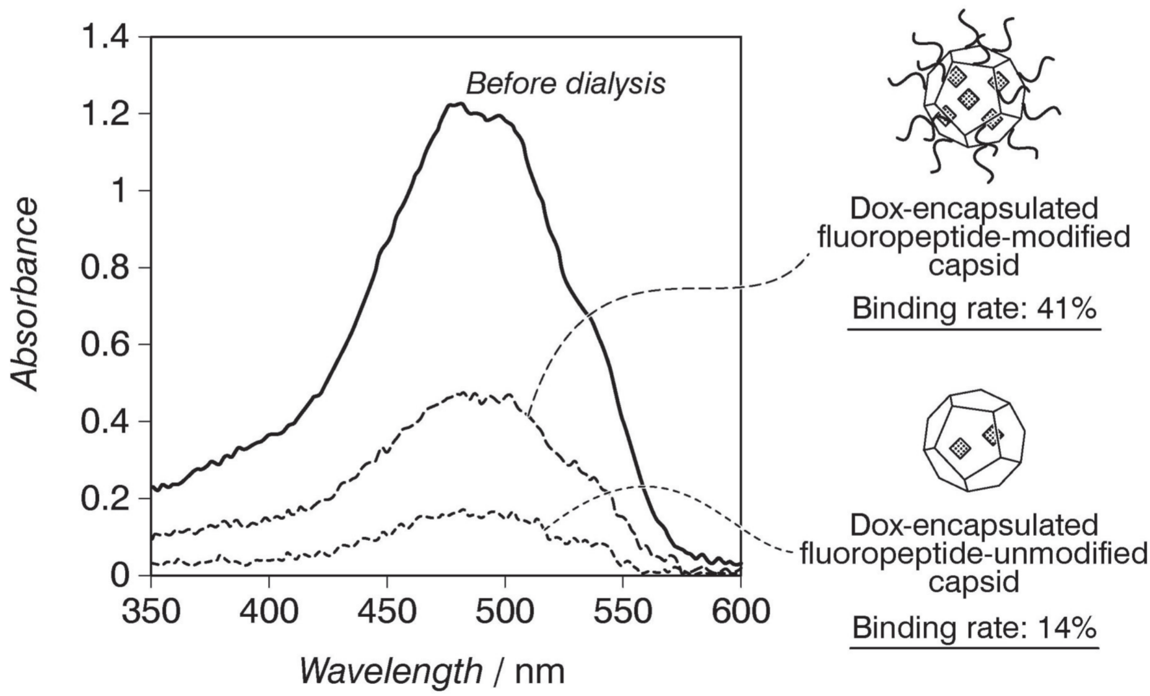

FIG. 18

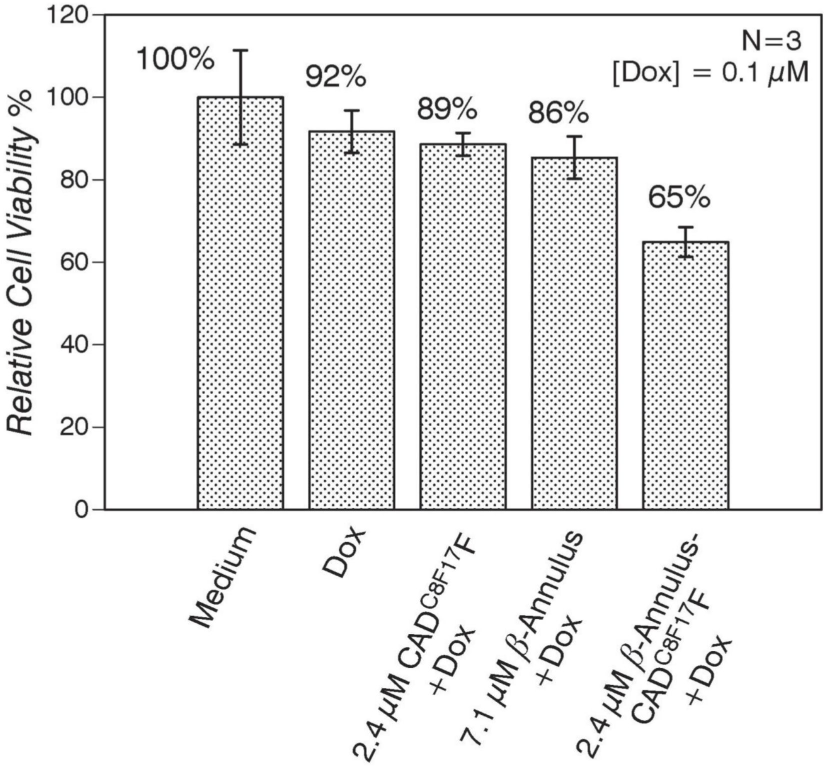
Relative Cell Viability %
120
100
80
60
40
20
0
N=3
[Dox] = 0.1 μM
100%
92%
89%
86%
65%
Medium
Dox
2.4 μM CAD^C8F17F +Dox
7.1 μM β-Annulus +Dox
2.4 μM β-Annulus-CAD^C8F17F +Dox

FIG. 19

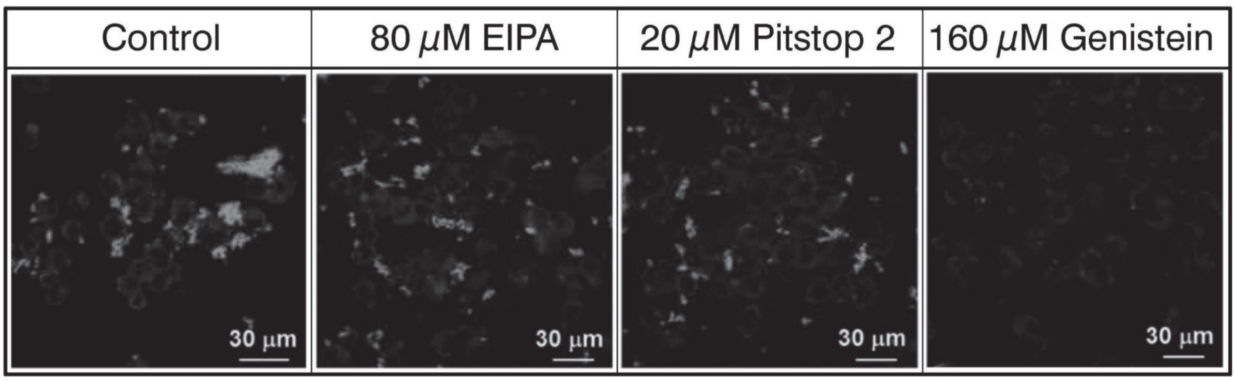
Control
80 μM EIPA
20 μM Pitstop 2
160 μM Genistein
30 μm
30 μm
30 μm
30 μm

FIG. 20

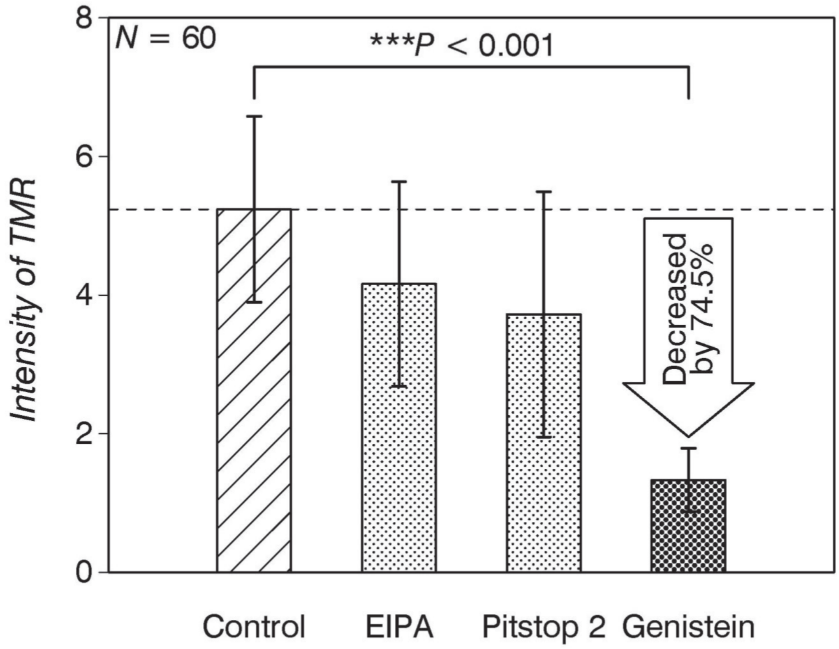
8
6
4
2
0
Intensity of TMR
N = 60
***P < 0.001
Decreased by 74.5%
Control
EIPA
Pitstop 2
Genistein

FIG. 21

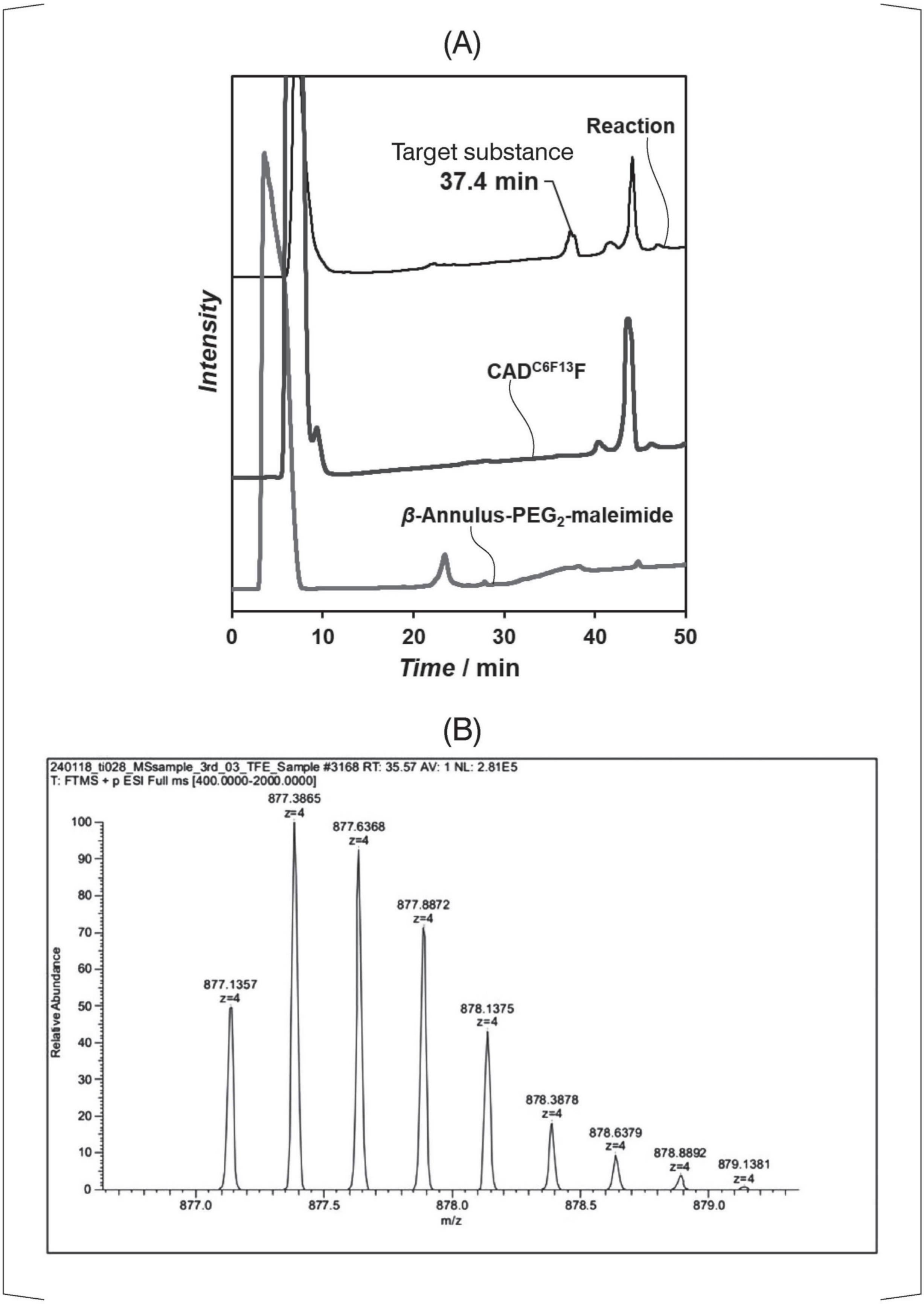
(A)
Reaction
Target substance
37.4 min
Intensity
CADC6F13F
β-Annulus-PEG2-maleimide
0
10
20
30
40
50
Time / min
(B)
240118_ti028_MSsample_3rd_03_TFE_Sample #3168 RT: 35.57 AV: 1 NL: 2.81E5
T: FTMS + p ESI Full ms [400.0000-2000.0000]
877.1357 z=4
877.3865 z=4
877.6368 z=4
877.8872 z=4
878.1375 z=4
878.3878 z=4
878.6379 z=4
878.8892 z=4
879.1381 z=4
Relative Abundance
m/z

FIG. 22

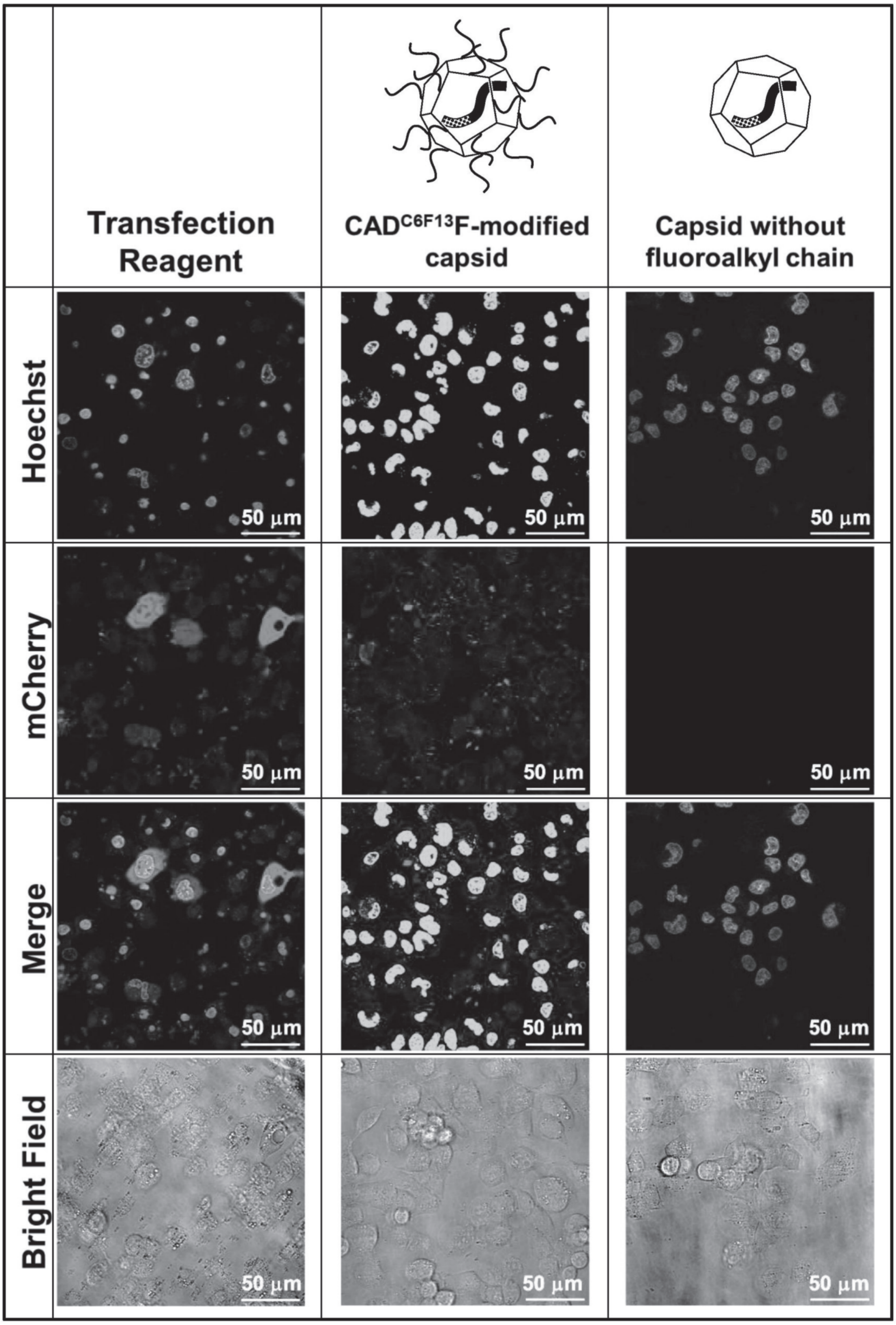
Transfection Reagent
CADC6F13F-modified capsid
Capsid without fluoroalkyl chain
Hoechst
mCherry
Merge
Bright Field
50 μm

*FIG. 23*

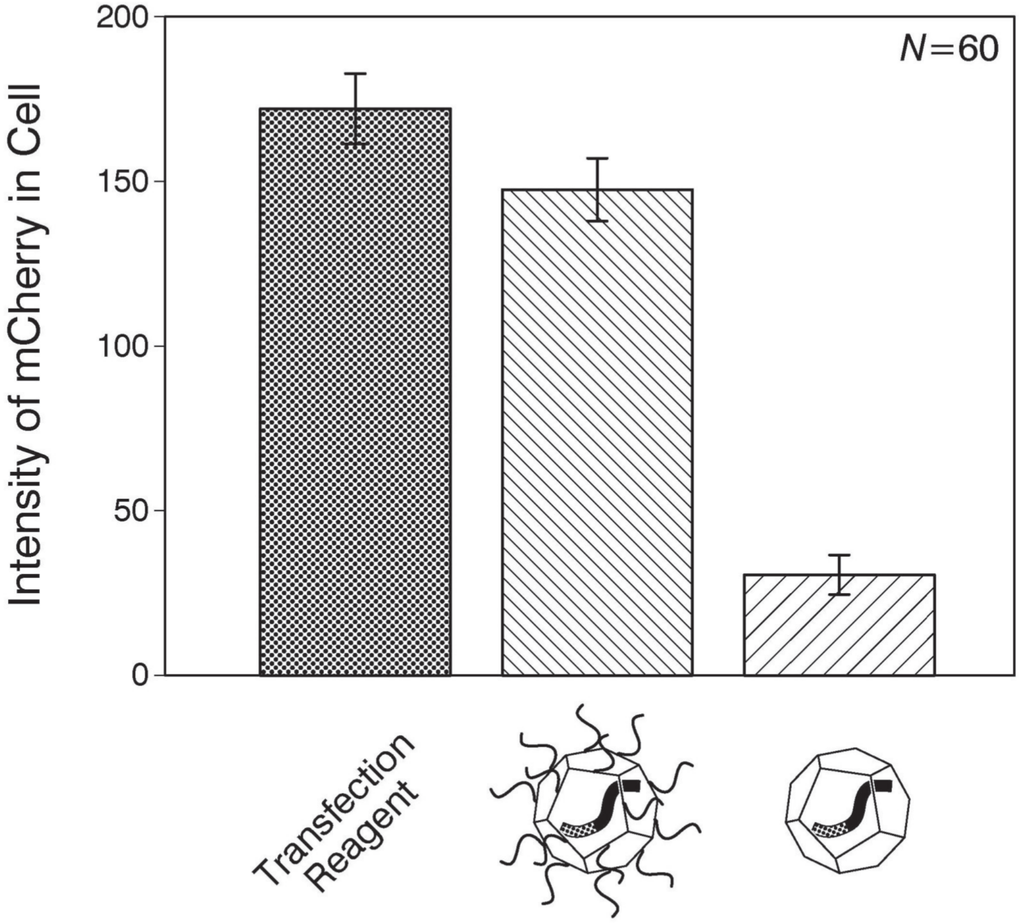
200
150
100
50
0
Intensity of mCherry in Cell
N=60
Transfection Reagent

**INTERNATIONAL SEARCH REPORT**

International application No. **PCT/JP2024/016503**

**A. CLASSIFICATION OF SUBJECT MATTER**

***C12N 7/00***(2006.01)i; ***A61K 9/51***(2006.01)i; ***A61K 35/76***(2015.01)i; ***A61P 35/00***(2006.01)i; *C12N 15/40*(2006.01)n
FI: C12N7/00 ZNA; A61K9/51; A61P35/00; A61K35/76; C12N15/40

According to International Patent Classification (IPC) or to both national classification and IPC

**B. FIELDS SEARCHED**

Minimum documentation searched (classification system followed by classification symbols)

C12N7/00; A61K9/51; A61K35/76; A61P35/00; C12N15/40

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | SAKAMOTO, Kentarou et al. Artificial Nanocage Formed via Self-Assembly of β-Annulus Peptide for Delivering Biofunctional Proteins into Cell Interiors. Bioconjugate Chemistry. 2022, vol. 33, no. 2, pp. 311-320, DOI: 10.1021/acs.bioconjchem.1c00534<br>in particular, abstract, p. 312, left column, lines 8-11, fig. 1, 3, 5 | 1-9 |
| Y | ZHANG, Zhenjing et al. The fluorination effect of fluoroamphiphiles in cytosolic protein delivery. Nature Communications. 2018, vol. 9, no. 1, 1377, pp. 1-8, DOI: 10.1038/s41467-01 8-037<br>in particular, fig. 1, 2, 5f | 1-9 |
| Y | XU, Jun et al. A general strategy towards personalized nanovaccines based on fluoropolymer s for post-surgical cancer immunotherapy. Nature Nanotechnology. 2020, vol. 15, no. 12, pp. 1043-1052, DOI: 10.1038/s41565-020-00781-4<br>in particular, p. 1043, left column, line 3 from the bottom to right column, line 1, p. 1045, right column, lines 42-46, fig. 1a, 2 | 1-9 |
| Y | WO 2023/048236 A1 (AGC INC.) 30 March 2023 (2023-03-30)<br>claims 1, 12, examples | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 July 2024** | **16 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/016503**

**C. DOCUMENTS CONSIDERED TO BE RELEVANT**

| Category* | Citation of document, with indication, where appropriate, of the relevant passages | Relevant to claim No. |
|---|---|---|
| Y | WO 2022/149584 A1 (AGC INC.) 14 July 2022 (2022-07-14)<br>claims 1, 5, examples | 1-9 |
| A | WO 2022/043449 A1 (KEMIJSKI INSTITUT) 03 March 2022 (2022-03-03)<br>claims | 1-9 |
| A | WO 2022/138637 A1 (EPSILON MOLECULAR ENG INC.) 30 June 2022 (2022-06-30)<br>claims | 1-9 |
| A | JP 2022-173834 A (UNIV TOTTORI) 22 November 2022 (2022-11-22)<br>claims | 1-9 |

Form PCT/ISA/210 (second sheet) (July 2022)

**INTERNATIONAL SEARCH REPORT**

International application No.
**PCT/JP2024/016503**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/016503**

| Patent document cited in search report | Publication date (day/month/year) | Patent family member(s) | Publication date (day/month/year) |
|---|---|---|---|
| WO 2023/048236 A1 | 30 March 2023 | CN 117999272 A | |
| WO 2022/149584 A1 | 14 July 2022 | (Family: none) | |
| WO 2022/043449 A1 | 03 March 2022 | LU 102016 B1 | |
| WO 2022/138637 A1 | 30 June 2022 | (Family: none) | |
| JP 2022-173834 A | 22 November 2022 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023076053 A
- JP 2022173834 A
- WO 2012130941 A
- WO 2021060506 A
- WO 2023048236 A


### Non-patent literature cited in the description


- *Chem. Commun.*, 2018, vol. 54, 8944
- *Bioconjugate Chem.*, 2019, vol. 30, 1636-1641
- *J. Org. Chem.*, 2020, vol. 85, 1668-1673
- *J Pept Sci.*, 2017, vol. 23, 636-643
- **KOSTRZEWA-NOWAK et al.** *British Journal of Cancer*, 2005, vol. 93, 89-97